(19) Europäisches Patentamt European Patent Office Office européen des brevets

(11) **EP 3 271 384 B1**

(12) **EUROPEAN PATENT SPECIFICATION**

(45) Date of publication and mention of the grant of the patent:
**26.07.2023 Bulletin 2023/30**

(21) Application number: **16719507.2**

(22) Date of filing: **17.03.2016**

(51) International Patent Classification (IPC):
**C07K 14/725** (2006.01)    **C12N 5/0783** (2010.01)

(52) Cooperative Patent Classification (CPC):
**C07K 14/7051; A61K 35/12; C12N 5/0636; C12N 9/1241;** C12N 2510/00

(86) International application number:
**PCT/IB2016/051510**

(87) International publication number:
**WO 2016/147145 (22.09.2016 Gazette 2016/38)**

(54) **GENIC SEQUENCE ENCODING A TCR SPECIFIC FOR THE HUMAN MHC CLASS I COMPLEX SPECIFIC FOR THE HLA-AO2 COMPLEX AND THE PEPTIDE HTERT865-873, AS WELL AS ITS USE IN THE ENGINEERING OF T LYMPHOCYTES FOR POSSIBILE CLINICAL APPLICATIONS OF ADOPTIVE TRANSFER**

GENSEQUENZ ZUR CODIERUNG EINES TCR, DAS FÜR DEN HUMANEN MHC-KLASSE-I-KOMPLEX SPEZIFISCH FÜR DEN HLA-AO2-KOMPLEX UND DAS PEPTID HTERT865-873 SPEZIFISCH IST, SOWIE DEREN VERWENDUNG ZUR HERSTELLUNG VON T-LYMPHOZYTEN FÜR MÖGLICHE KLINISCHE ANWENDUNGEN DES ADOPTIVEN TRANSFER

SÉQUENCE GÉNIQUE CODANT POUR UN TCR SPÉCIFIQUE DU CMH HUMAIN DE CLASSE I POUR LE COMPLEXE HLA-AO2 ET LE PEPTIDE HTERT865-873, AINSI QUE SON UTILISATION DANS L'INGÉNIERIE DES LYMPHOCYTES T POUR DE POSSIBLES APPLICATIONS CLINIQUES DE TRANSFERT ADOPTIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.03.2015 IT PD20150062**

(43) Date of publication of application:
**24.01.2018 Bulletin 2018/04**

(73) Proprietor: **Università Degli Studi Di Verona 37129 Verona (IT)**

(72) Inventors:
• **UGEL, Stefano
  I-31024 Ormelle (Treviso) (IT)**
• **BRONTE, Vincenzo
  I-35031 Abano Terme (Padova) (IT)**
• **SANDRI, Sara
  I-37015 S. Ambrogio Valpolicella (Verona) (IT)**
• **BOBISSE, Sara
  CH-1066 Epalinges (CH)**

(74) Representative: **Croce, Valeria Jacobacci & Partners S.p.A. Piazza Mario Saggin, 2 35131 Padova (IT)**

(56) References cited:
**WO-A1-2005/116646    WO-A2-2013/056211**

• **ZHONG-LI LIAO ET AL: "Antitumor effect of new multiple antigen peptide based on HLA-A0201-restricted CTL epitopes of human telomerase reverse transcriptase (hTERT)", CANCER SCIENCE, vol. 103, no. 11, 28 November 2012 (2012-11-28), pages 1920-1928, XP055228164, JP ISSN: 1347-9032, DOI: 10.1111/j.1349-7006.2012.02410.x**

- **BRUNO LAUGEL ET AL: "Different T Cell Receptor Affinity Thresholds and CD8 Coreceptor Dependence Govern Cytotoxic T Lymphocyte Activation and Tetramer Binding Properties", JOURNAL OF BIOLOGICAL CHEMISTRY, vol. 282, no. 33, 17 August 2007 (2007-08-17), pages 23799-23810, XP055228165, US ISSN: 0021-9258, DOI: 10.1074/jbc.M700976200**
- **S. UGEL ET AL: "Autoimmune B-cell lymphopenia after successful adoptive therapy with telomerase-specific T lymphocytes", BLOOD, vol. 115, no. 7, 18 February 2010 (2010-02-18), pages 1374-1384, XP055228169, US ISSN: 0006-4971, DOI: 10.1182/blood-2009-07-233270**
- **S. SANDRI ET AL: "Feasibility of Telomerase-Specific Adoptive T-cell Therapy for B-cell Chronic Lymphocytic Leukemia and Solid Malignancies", CANCER RESEARCH, vol. 76, no. 9, 1 May 2016 (2016-05-01), pages 2540-2551, XP055285966, US ISSN: 0008-5472, DOI: 10.1158/0008-5472.CAN-15-2318**

Remarks:
The complete document including Reference Table(s) and the Sequence Listing(s) can be downloaded from the EPO website

**Description**

Telomerase (TERT) is the reverse transcriptase mainly implicated in the lengthening of telomeres in mammalian cells and is an important universal tumour antigen; in fact, TERT is expressed in more than 85% of tumour cells, regardless of the origin or histologic type. WO2005/116646 describes in example 2 a method for generating TCR's directed against the complex of HLA-A02 presenting the hTERT865-873 peptide by creating a library of TCR's, that are then screened using ELISA.

OBJECT OF THE INVENTION

A first object of the present invention is therefore to provide the sequences encoding the $\alpha$ and $\beta$ chains of the transgenic TCR specific for the MHC class I/peptide $hTERT_{865-873}$ complex restricted for HLA-A2.

In another object, the invention describes a construct comprising such sequences and a vector comprising such a construct.

The sequences of the specific $\alpha5$ and $\beta3$ subunits of the TCR are also provided.

The isolated lymphocyte cells transduced with the vector described are a further object of the invention.

In a still further object, the use of T lymphocytes obtained for the diagnosis and treatment of tumour diseases in humans and animals is described.

BRIEF DESCRIPTION OF THE FIGURES

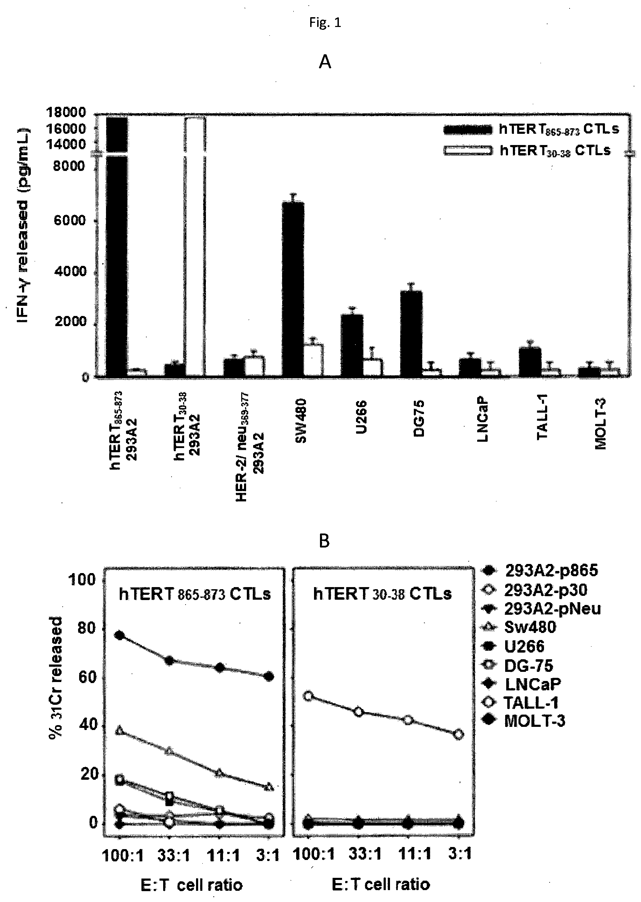

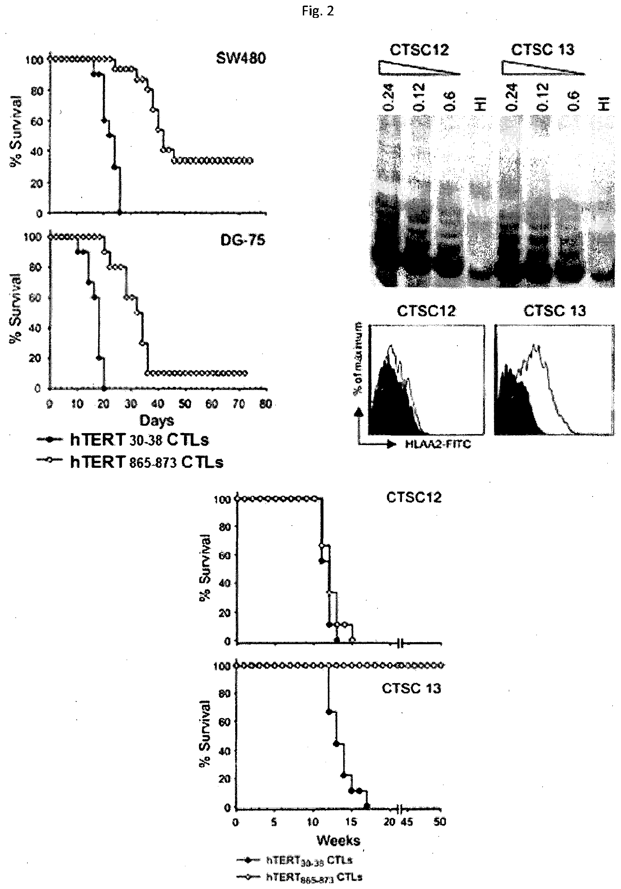

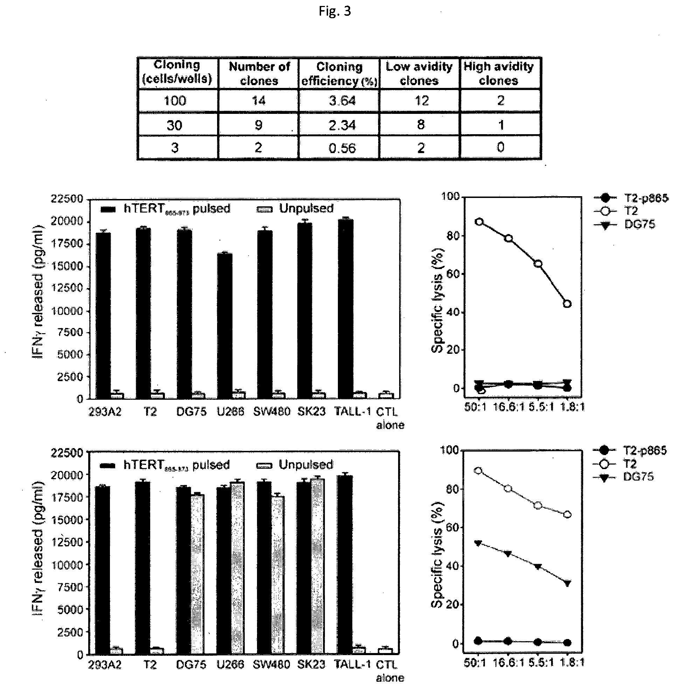

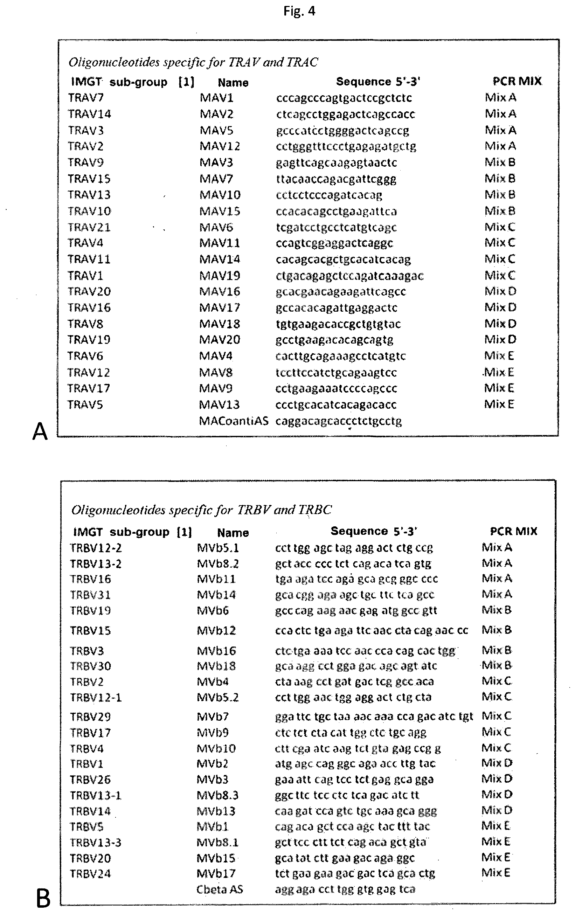

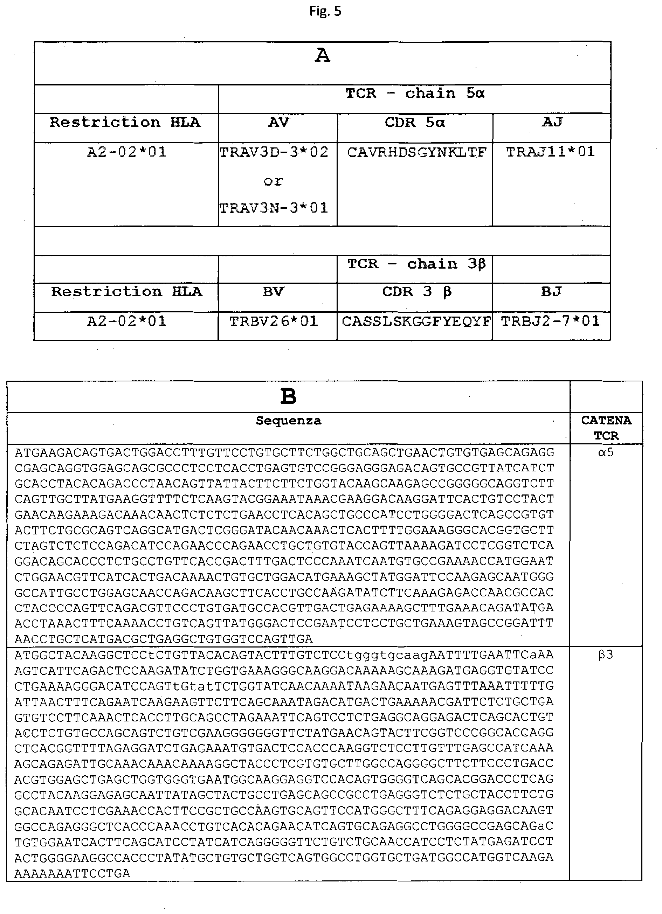

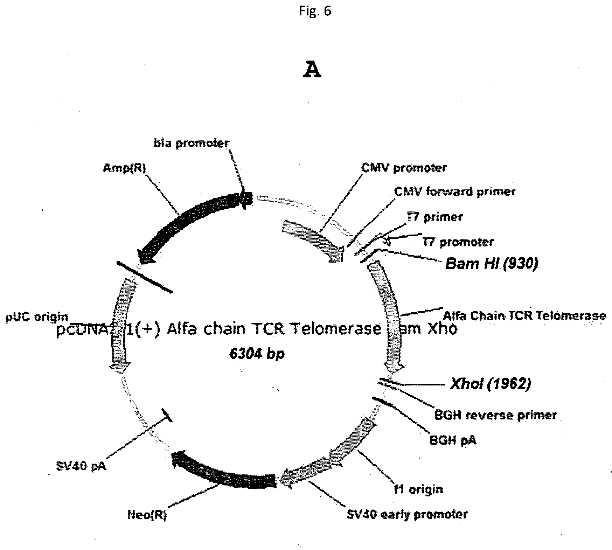

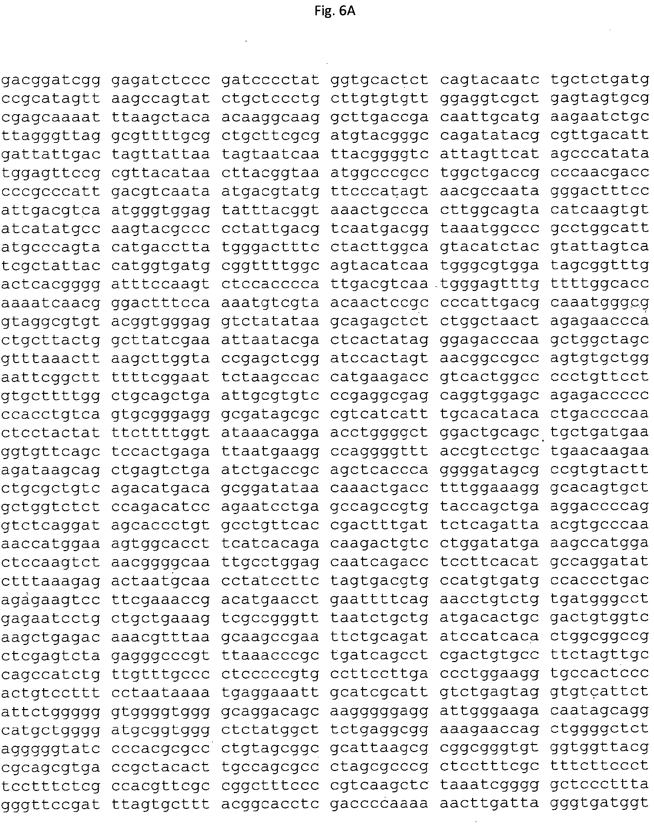

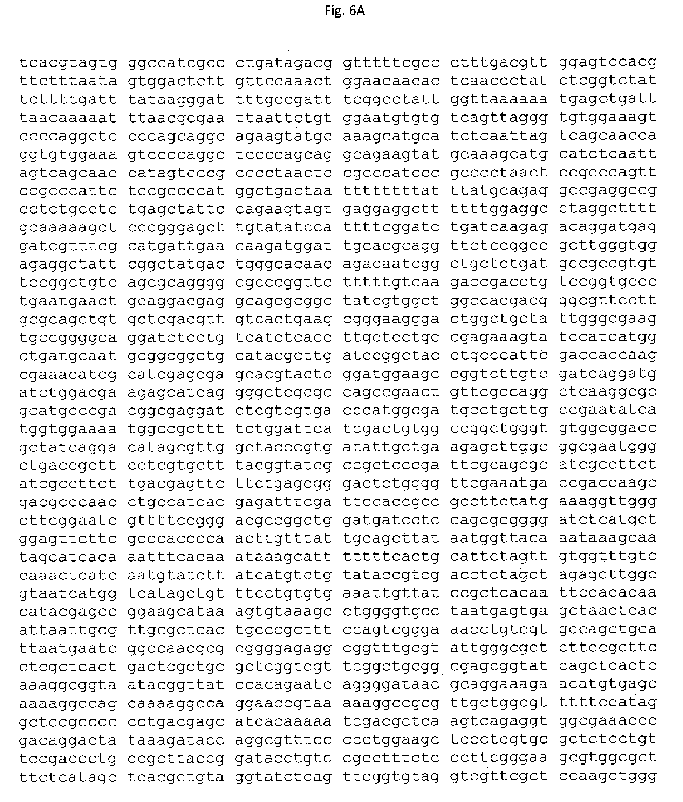

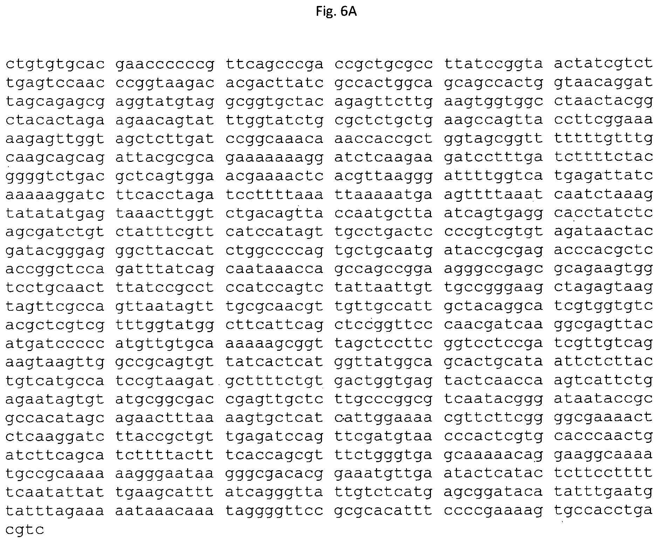

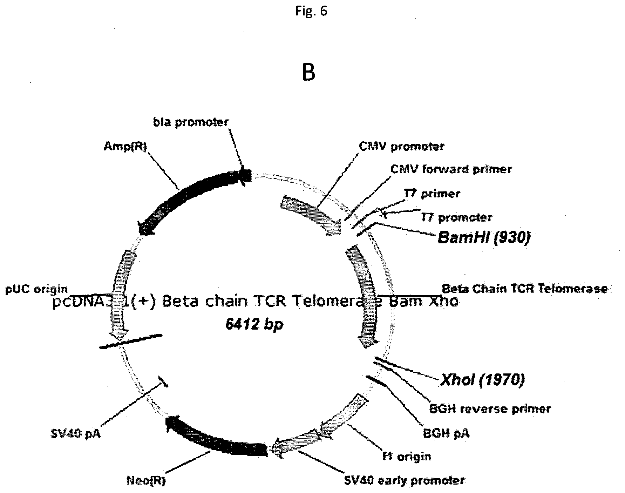

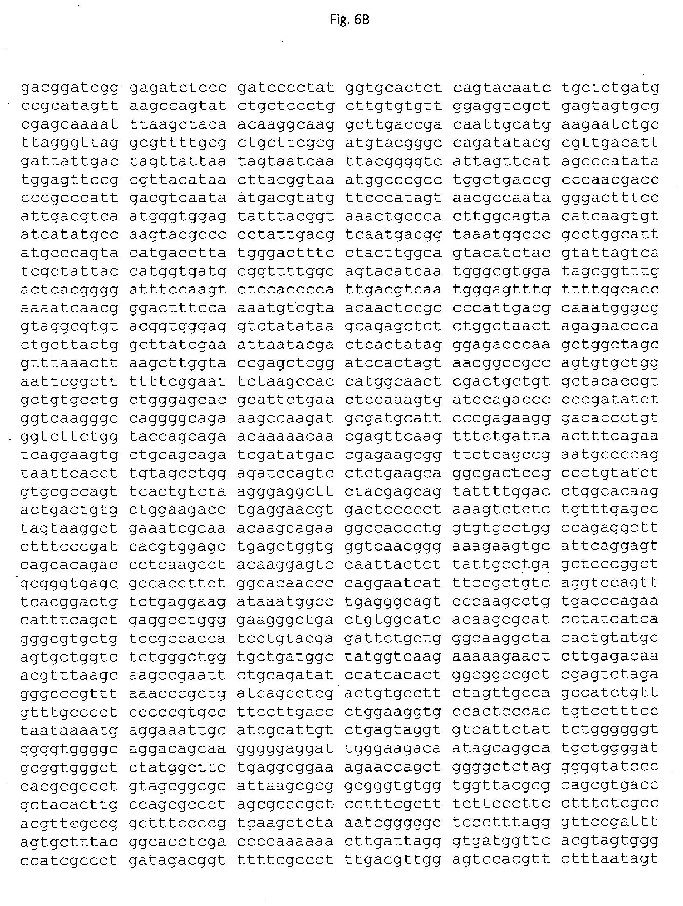

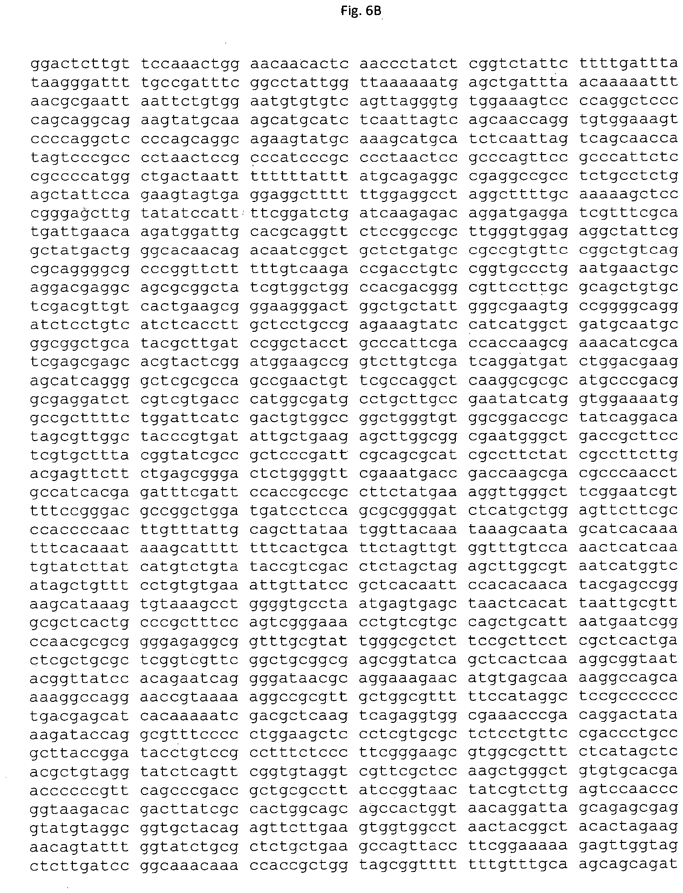

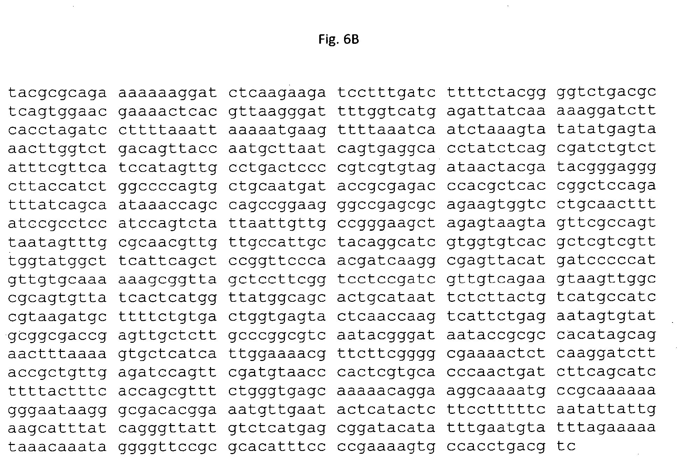

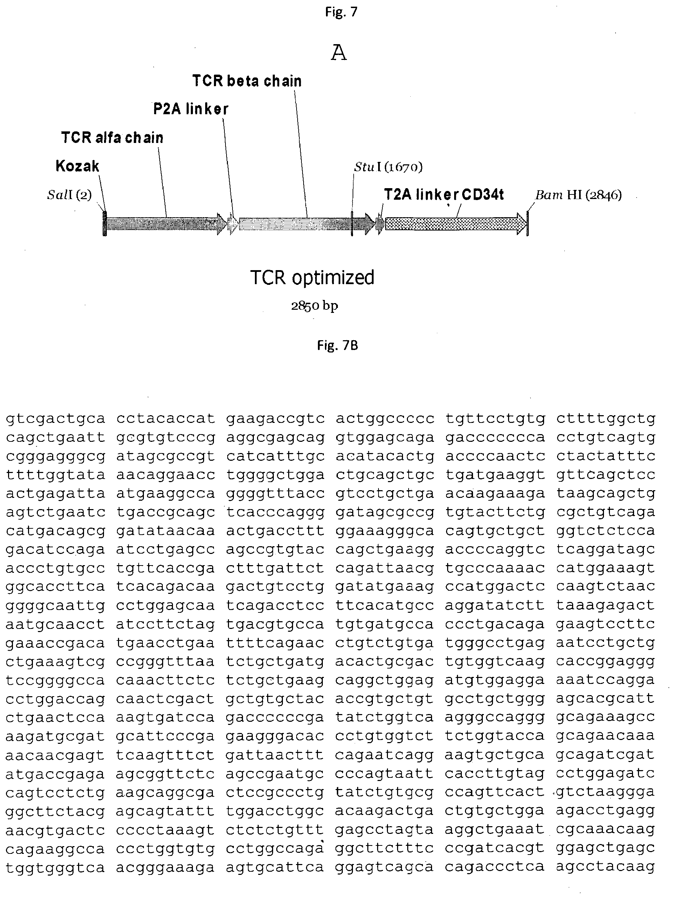

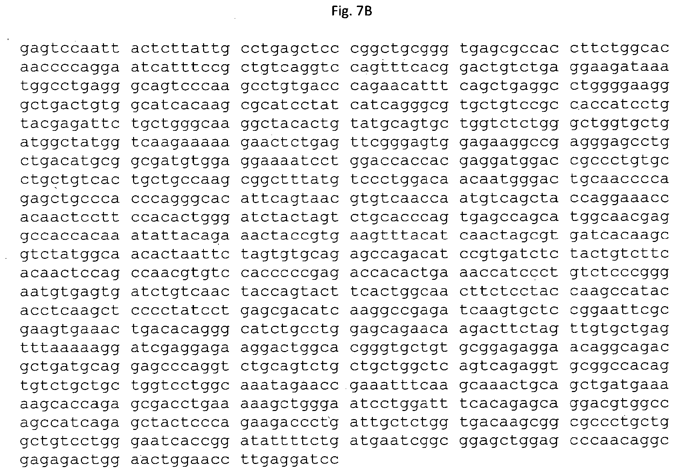

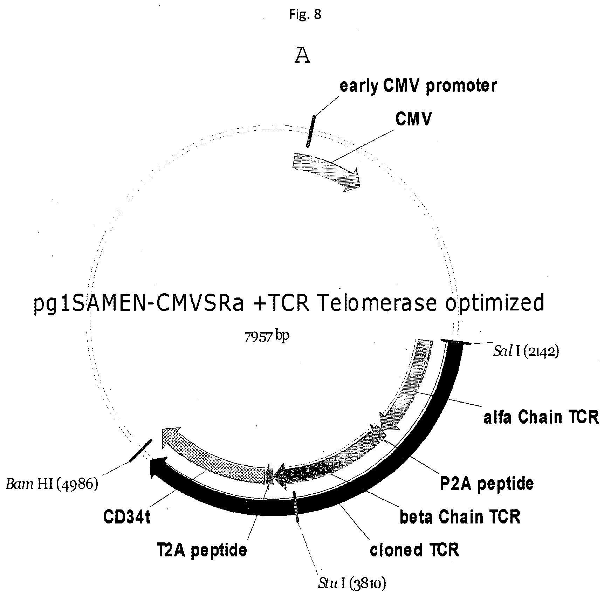

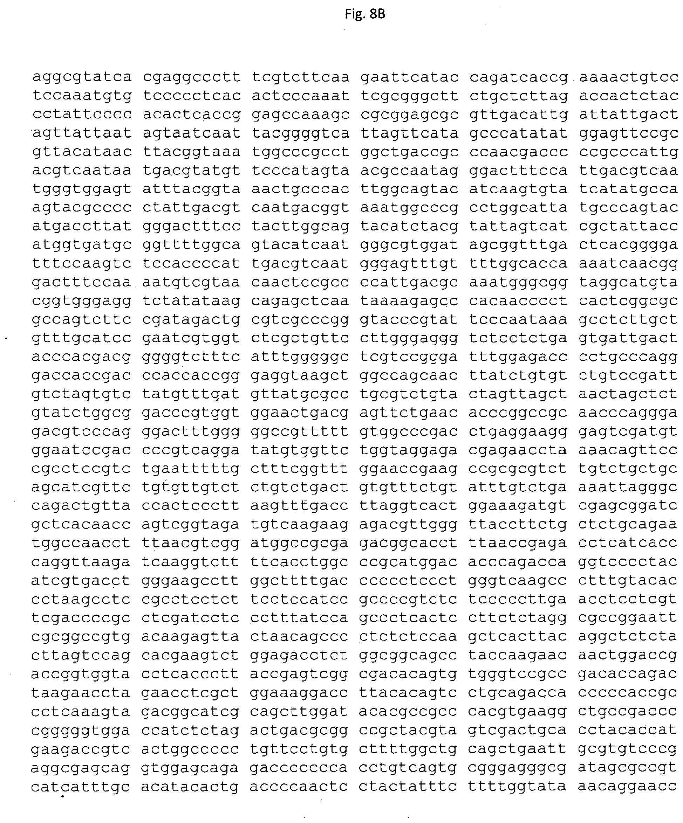

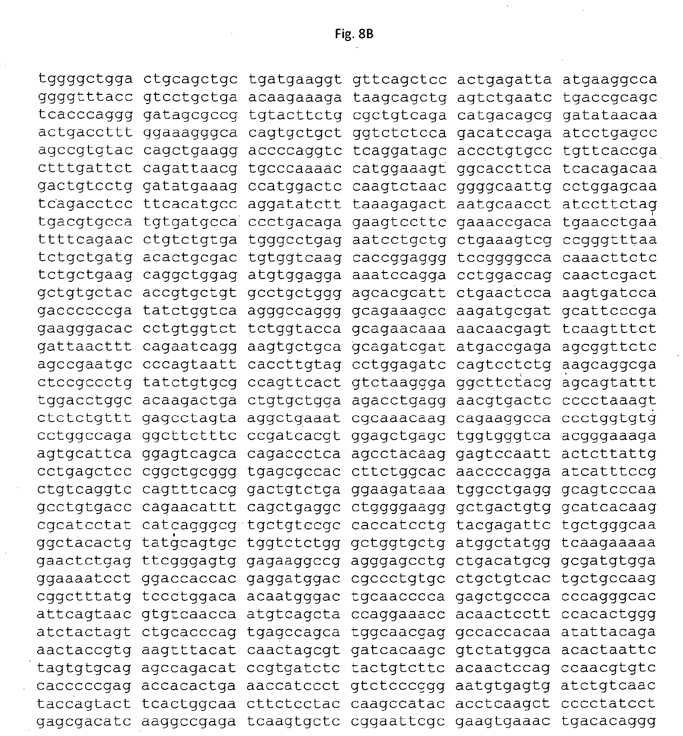

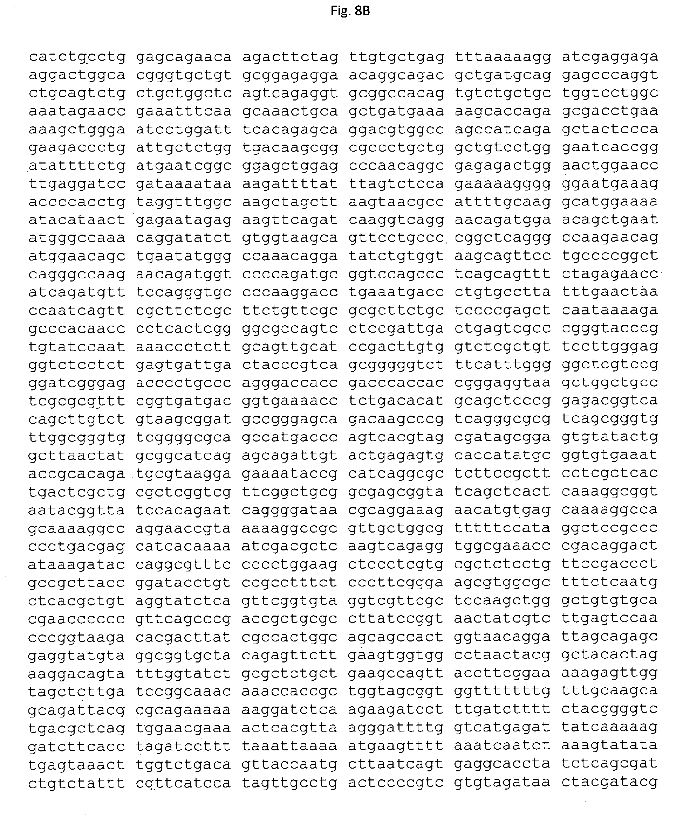

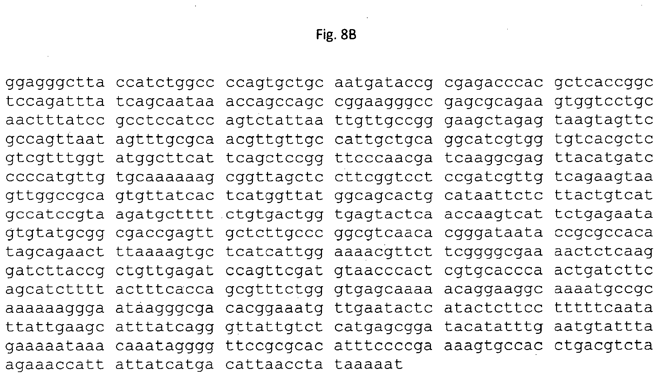

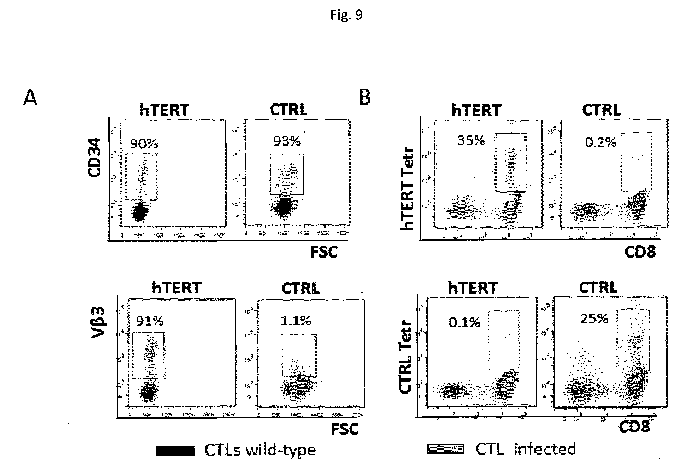

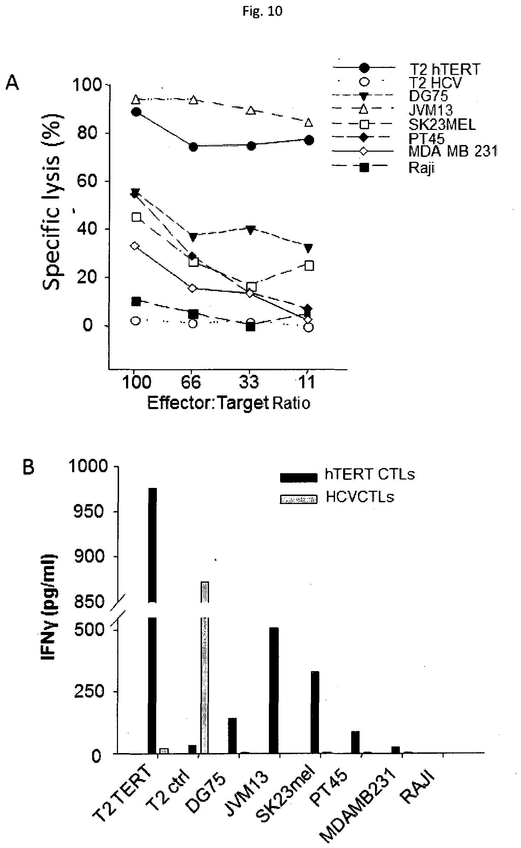

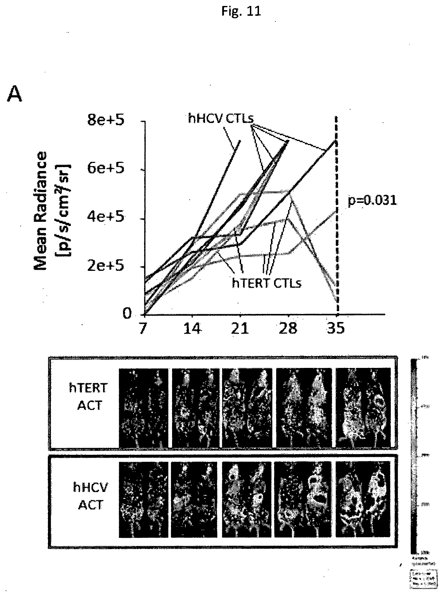

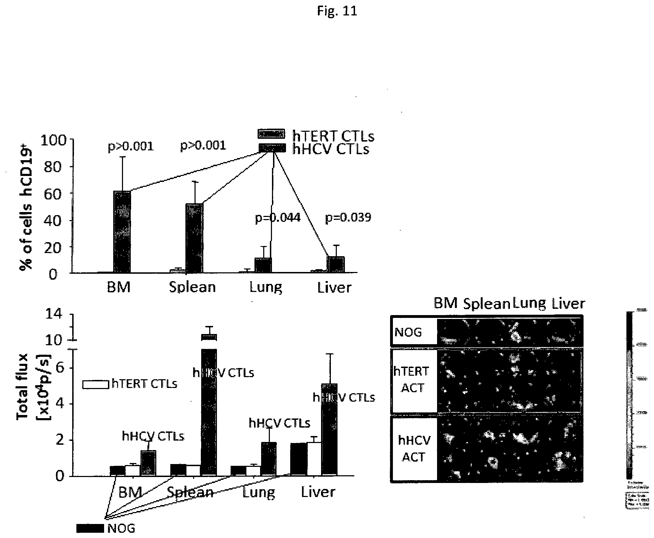

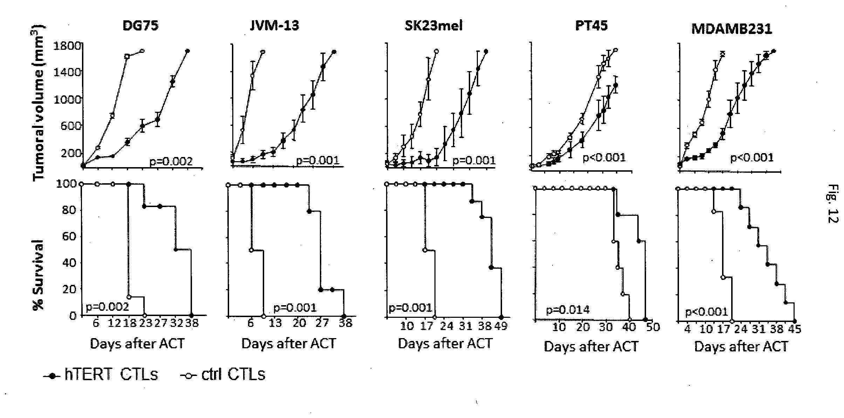

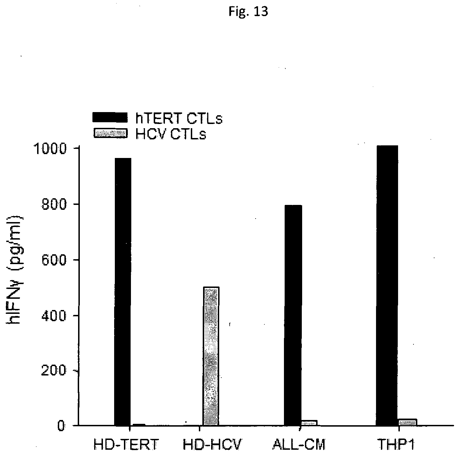

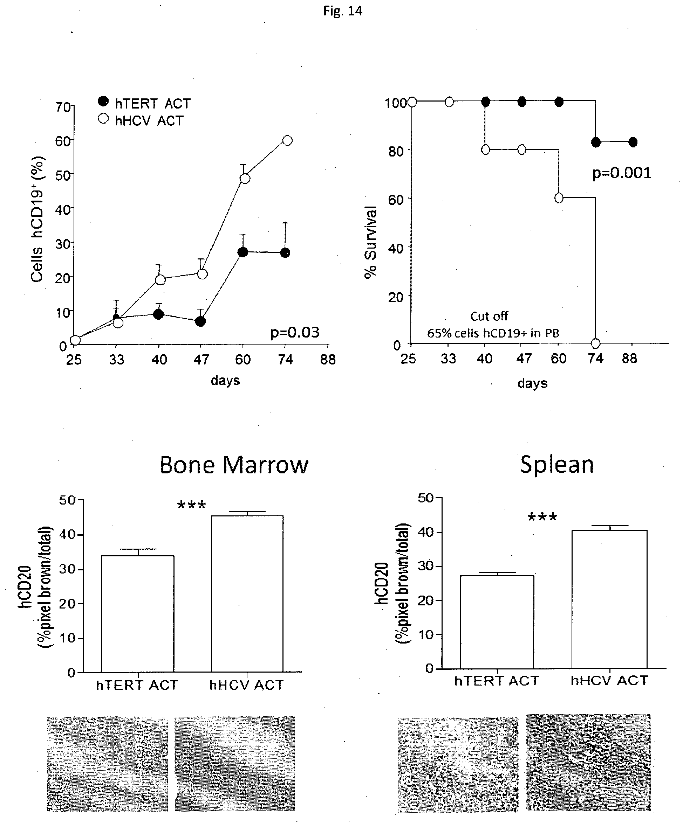

Figure 1 shows the results of functional release assays of IFN$\gamma$ (A) and chromium (B); Figure 2 shows the results of the adoptive transfer of T lymphocytes specific for TERT to verify the ability to contrast *in vivo* tumour growth in immunodeficient animals inoculated with immortalised cells (SW480 and DG75); Figure 3 shows the data for the isolated clones and their avidity measured by functional release assays of IFN$\gamma$ and chromium; Figure 4 shows the *primers* specific for the $\alpha$ (A) and $\beta$ (B) chains of the TCR used for the identification of the CDR3 regions; Figure 5 shows the results of the IMGT (A) analysis that led to the identification of the specific subunits of the TCR (as and $\beta3$) and the complete sequences; Figure 6 shows the pcDNA3.1 vectors and the sequences containing the $\alpha$ (6A) and $\beta$ (6B) chains of the TCR specific for $hTERT_{865-673}$; Figure 7 shows the construct (A) optimised for the expression in the retroviral vector, composed of the sequences of the $\alpha$ and $\beta$ chains of the TCR specific for $hTERT_{865-873}$, from two cleavage sites and the coding sequence for the truncated form of CD34 (and related sequence) and the related sequence (B); Figure 8 shows the retroviral vector containing the construct (A) optimised for the expression of the $\alpha$ and $\beta$ chains of the TCR specific for $hTERT_{865-873}$ and the related sequence (B); Figure 9 shows the results of the infection assays of human PBMC by means of retroviral vector that generates a population of T lymphocytes expressing the correct chain of the TCR specific for $hTERT_{865-873}$ (A) and able to specifically recognise the tetramer $hTERT_{865-873}$ (B); Figure 10 shows the results of the functional assays (specific lysis, 10A and release of IFN$\gamma$, 10B) of PBMCs expressing the TCR specific for $hTERT_{865-873}$ that are able to recognise *in vitro* immortalised cancer cells expressing high levels of telomerase activity; Figure 11 shows the results of adoptive transfer *in vivo* of the lymphocytes specific for $hTERT_{865-873}$ that are able to control the spread of the leukemic tumour cell line in immunodeficient mice; Figure 12 shows the lymphocytes specific for the peptide $hTERT_{865-873}$ that are able to control the growth *in vivo* of tumours of different histological origin; Figure 13 shows the results of the functional assay (ELISA for IFN-$\gamma$) of transduced PBMCs specific for $hTERT_{865-873}$ that are able to recognise *in vitro* acute lymphoblastic leukaemia and acute myeloid leukaemia lines; Figure 14 shows the results of the treatment *in vivo* of animals with acute lymphoblastic leukaemia (ALL-CM-hTERT: n=8; ctrl CTLs: n=6).

DETAILED DESCRIPTION OF THE INVENTION

According to an object of the invention, the sequences encoding the $\alpha$ and $\beta$ chains of the transgenic TCR specific for the MHC class I/peptide $hTERT_{865-873}$ complex restricted to HLA-A2 are described.

In particular, these sequences are:

| SEQ. ID. Ref. | Sequence | TCR CHAIN |
|---|---|---|
| SEQ. ID. no. 1 | ATGAAGACAGTGACTGGACCTTTGTTCCTGT GCTTCTGGCTGCAGCTGAACTGTGTGAGCAG AGGCGAGCAGGTGGAGCAGCGCCCTCCTCAC CTGAGTGTCCGGGAGGGAGACAGTGCCGTTA TCATCTGCACCTACACAGACCCTAACAGTTA TTACTTCTTCTGGTACAAGCAAGAGCCGGGG GCAGGTCTTCAGTTGCTTATGAAGGTTTTCT CAAGTACGGAAATAAACGAAGGACAAGGATT CACTGTCCTACTGAACAAGAAAGACAAACAA CTCTCTCTGAACCTCACAGCTGCCCATCCTG GGGACTCAGCCGTGTACTTCTGCGCAGTCAG GCATGACTCGGGATACAACAAACTCACTTTT GGAAAGGGCACGGTGCTTCTAGTCTCTCCAG ACATCCAGAACCCAGAACCTGCTGTGTACCA GTTAAAAGATCCTCGGTCTCAGGACAGCACC CTCTGCCTGTTCACCGACTTTGACTCCCAAA TCAATGTGCCGAAAACCATGGAATCTGGAAC GTTCATCACTGACAAAACTGTGCTGGACATG AAAGCTATGGATTCCAAGAGCAATGGGGCCA | α5 |
| | TTGCCTGGAGCAACCAGACAAGCTTCACCTG CCAAGATATCTTCAAAGAGACCAACGCCACC TACCCCAGTTCAGACGTTCCCTGTGATGCCA CGTTGACTGAGAAAGCTTTGAAACAGATAT GAACCTAAACTTTCAAAACCTGTCAGTTATG GGACTCCGAATCCTCCTGCTGAAAGTAGCCG GATTTAACCTGCTCATGACGCTGAGGCTGTG GTCCAGTTGA | |

(continued)

| SEQ. ID. Ref. | Sequence | TCR CHAIN |
|---|---|---|
| SEQ. ID. no. 2 | ATGGCTACAAGGCTCCtCTGTTACACAGTAC TTTGTCTCCtgggtgcaagAATTTTGAATTC aAAAGTCATTCAGACTCCAAGATATCTGGTG AAAGGGCAAGGACAAAAAGCAAAGATGAGGT GTATCCCTGAAAAGGGACATCCAGTtGtatT CTGGTATCAACAAAATAAGAACAATGAGTTT AAATTTTTGATTAACTTTCAGAATCAAGAAG TTCTTCAGCAAATAGACATGACTGAAAAACG ATTCTCTGCTGAGTGTCCTTCAAACTCACCT TGCAGCCTAGAAATTCAGTCCTCTGAGGCAG GAGACTCAGCACTGTACCTCTGTGCCAGCAG TCTGTCGAAGGGGGGGTTCTATGAACAGTAC TTCGGTCCCGGCACCAGGCTCACGGTTTTAG AGGATCTGAGAAATGTGACTCCACCCAAGGT CTCCTTGTTTGAGCCATCAAAAGCAGAGATT GCAAACAAACAAAAGGCTACCCTCGTGTGCT TGGCCAGGGGCTTCTTCCCTGACCACGTGGA GCTGAGCTGGTGGGTGAATGGCAAGGAGGTC CACAGTGGGGTCAGCACGGACCCTCAGGCCT ACAAGGAGAGCAATTATAGCTACTGCCTGAG CAGCCGCCTGAGGGTCTCTGCTACCTTCTGG CACAATCCTCGAAACCACTTCCGCTGCCAAG TGCAGTTCCATGGGCTTTCAGAGGAGGACAA GTGGCCAGAGGGCTCACCCAAACCTGTCACA CAGAACATCAGTGCAGAGGCCTGGGGCCGAG CAGaCTGTGGAATCACTTCAGCATCCTATCA TCAGGGGGTTCTGTCTGCAACCATCCTCTAT GAGATCCTACTGGGGAAGGCCACCCTATATG CTGTGCTGGTCAGTGGCCTGGTGCTGATGGC CATGGTCAAGAAAAAAATTCCTGA | β3 |

In the context of the present invention, the sequences homologous to those discussed above are equally contemplated.

"Homologous" means those nucleotide sequences that, by virtue of the redundancy of the genetic code, encode for the same sequence.

As regards the specific α5 and β3 subunits of the TCR, these are:

| | TCR - 5α chain | | |
|---|---|---|---|
| HLA restriction | AV | CDR 5α | AJ |
| A2-02*01 | TRAV3D-3*02 or TRAV3N-3*01 | CAVRHDSGYNKLTF | TRAJ11*01 |

| | TCR - 3β chain | | |
|---|---|---|---|
| HLA restriction | BV | CDR 3 β | BJ |
| A2-02*01 | TRBV26*01 | CASSLSKGGFYEQYF | TRBJ2-7*01 |

According to a further object, the use of the sequences of the invention for the preparation of a construct useful in the preparation of a transfection vector is described.

The construct, as well as the vector comprising such a construct, is a further object of the invention.

In a preferred aspect of the invention, such a construct is represented by a plasmid for the generation of viral vectors.

In particular, this construct comprises the sequences encoding the $\alpha$ and $\beta$ chains of the transgenic TCR specific for the MHC class I/peptide hTERT$_{865-873}$ complex restricted for HLA-A2; these sequences preferably correspond to the SEQ. ID. no. 1 and 2.

In a preferred aspect, the construct further comprises a sequence encoding the truncated form of the CD34 molecule having a marker function. This sequence is useful for identifying transgenic cells.

According to the present invention, these viral vectors are preferably retroviruses or lentiviruses.

The person skilled in the art will be able to prepare the necessary modifications to adapt the construct to the selected vector, based on the purposes to achieve.

Isolated cells infected with the vector described are further objects of the present invention.

In a still further aspect, the invention describes isolated T lymphocytes transduced with the use of the sequences described above.

Preferably, these lymphocytes are lymphocytes of a mammal and more preferably are human lymphocytes.

In a particular aspect of the invention, such isolated transduced T lymphocytes can be used for the treatment or diagnosis of neoplastic diseases.

In a preferred aspect, the treatment of neoplastic diseases includes the adoptive transfer technique.

Such neoplastic diseases include solid tumours of different histology and haematic neoplasias.

More specifically, the tumours tested include malignant neoplasias such as: Burkitt's lymphoma, chronic B - B CLL lymphocytic leukaemia, melanoma, pancreatic adenocarcinoma, breast cancer, colon cancer, acute lymphoblastic leukaemia (ALL) and acute myeloid leukaemia (AML).

*Experimental part*

Immunisation of HHD mice to obtain T lymphocytes specific for the peptide hTERT$_{865-873}$

By immunising transgenic mice expressing the human leukocyte antigen (HLA)-A2 (HHD mice), a population of T lymphocytes was isolated expressing the T lymphocyte receptor (TCR) with high affinity for the peptide hTERT$_{865-873}$. The immunisation was carried out by inoculation of plasmid DNA coding for (h)TERT-LTB, cloned in the vector pVIJnsA (GENEART). The sequence was optimised to produce an inactive version of the enzyme telomerase, inserting two point mutations, D712A and V713I. After 15 days, the mice were sacrificed and leukocyte cultures stimulated by peptide were set up with the splenocytes. $5 \times 10^6$ splenocytes were plated in 24-well plates in 10% DMEM medium, supplemented with the specific peptide hTERT$_{865-873}$ (RLVDDFLLV corresponding to SEQ. ID. no .7) 0.1 $\mu$M. After 5 days, the specific recognition of the BULK of cells derived from HHD mice pulsed for 1 hour with the specific peptide hTERT$_{865-873}$ or with an unrelated control peptide was tested. After 24 hours of co-culture, the levels of IFN$\gamma$ secreted in the supernatant were assessed by ELISA (*Enzyme Linked ImmunoSorbent Assay*). In short, this assay consists in transferring the culture medium in a specific 96-well plate previously incubated with purified antibody specific for IFN$\gamma$. In some wells, in place of the sample, the calibration curve was plated using 1:2 scalar dilutions of the cytokine in question. After 2 hours of incubation at 37 °C, the plate was marked with the biotinylated anti-IFN$\gamma$ antibody for 1 hour, then followed by 30 minutes of incubation at room temperature, with streptavidin bound to peroxidase. The peroxidase reaction with its substrate (TMB - 3,3',5,5'-tetramethylbenzidine) allows the quantification of the cytokine of interest by reading at 450 nm. The functional activity of the polyclonal culture was further tested with a cytotoxicity assay based on the release of $^{51}$Cr. The clones were cultured for 5 hours with the human HLA-A2$^+$ T2 line, pulsed or not with the related peptide (5-10$\times 10^6$ of cells/ml of 10% DMEM medium with 5 $\mu$g/ml of peptide), or with immortalised malignant human lines, always positive for HLA-A2; apart from the MOLT-3 line that appears to be HLA-A2 negative. The target cells ($1 \times 10^6$) were marked, in pellet, with 100 pCi Na$_2$$^{51}$CrO$_4$ for 1 h at 37 °C. At the end of marking, the cells were suitably washed, and then resuspended in 5% RPMI medium, at a concentration of $2 \times 10^4$/ml; 100 $\mu$l of this cell suspension were seeded in a 96-well microplate with "U" bottom in the presence of scalar dilutions of effector cells, which were also distributed in 100 $\mu$l and in triplicate. After 5 hours of incubation, the supernatant was transferred into microplates containing a solid scintillant. The radioactivity emitted was measured with a $\gamma$ scintillation counter for plates. The specific lysis percentage was calculated using the following formula:

$$\% \text{ of specific release} = 100.x \frac{(Rsper - Rspon)}{(RMax - Rspon)}$$

wherein:

**Rspon** = spontaneous release, i.e. the amount of isotope released by the target cells incubated in the absence of effectors;

**Rmax** = maximum release of radioactivity obtained after repeated thawing of the radio-marked cells;

**Rsper** = experimental release measured for each ratio between effector/target, as can be seen in Figure 1 (Ugel S, Scarselli E, Iezzi M, Mennuni C, Pannellini T, Calvaruso F, Cipriani B, De Palma R, Ricci-Vitiani L, Peranzoni E, Musiani P, Zanovello P, Bronte V. Autoimmune B-cell lymphopenia after successful adoptive therapy with telomerase-specific T lymphocytes. Blood. 2010).

Given the specificity for the antigen, $2 \times 10^5$ specific polyclonal lymphocytes were cultured with $5 \times 10^6$ HHD splenocytes irradiated and pulsed with hTERT peptide$_{865-873}$, in 24-well plates, in the presence of interleukin (IL)-2 at a concentration of 20 IU/ml (Ugel S, Scarselli E, Iezzi M, Mennuni C, Pannellini T, Calvaruso F, Cipriani B, De Palma R, Ricci-Vitiani L, Peranzoni E, Musiani P, Zanovello P, Bronte V. Autoimmune B-cell lymphopenia after successful adoptive therapy with telomerase-specific T lymphocytes. Blood. 2010). This polyclonal lymphocyte line was also tested for the ability to contrast *in vivo* tumour growth in immunodeficient animals inoculated with immortalised cells (SW480 and DG75) or cancer stem cells isolated from colon cancer patient, if restricted for HLA-A2 haplotype (CTSC 13 line). See Figure 2 (Ugel S, Scarselli E, Iezzi M, Mennuni C, Pannellini T, Calvaruso F, Cipriani B, De Palma R, Ricci-Vitiani L, Peranzoni E, Musiani P, Zanovello P, Bronte V. Autoimmune B-cell lymphopenia after successful adoptive therapy with telomerase-specific T lymphocytes. Blood. 2010).

Cloning of a clone with high affinity for the peptide hTERT$_{865-873}$ by serial limit dilution

After 3 weekly re-stimulations, the polyclonal T lymphocytes obtained were cloned at different dilutions (3, 30 and 100 cells per well) and affinity for the antigen was assessed by quantification of the IFN$\gamma$ released in culture, as previously explained, or by cytotoxicity assay for chromium release (as explained below). While the most extreme dilution generated only 2 clones with low avidity and no high avidity clone, the other allowed obtaining 8 clones with low affinity and 1 clone with high affinity, defined as clone hTERT20.10. While the least extreme dilution (cloning to 100 cells) allowed isolating 2 clones with high avidity and 12 with low avidity, as shown in Figure 3.

This data indicates the low frequency of high avidity cells present in the polyclonal culture. Therefore, subsequent re-stimulations *in vitro* of the polyclonal culture determine a progressive loss of recognition by the lymphocytes of the antigenic targets given the expansion of low-avidity populations for the antigen, as evidenced by the letter by Maria Parkhurst in response to the article Ugel S, Scarselli E, Iezzi M, Mennuni C, Pannellini T, Calvaruso F, Cipriani B, De Palma R, Ricci-Vitiani L, Peranzoni E, Musiani P, Zanovello P, Bronte V. Autoimmune B-cell lymphopenia after successful adoptive therapy with telomerase-specific T lymphocytes. Blood. 2010 (http://www.bloodjournal.org/content/115/7/1374.e-letters).

However, being able to clone the T lymphocytes after several steps, one is able to overcome the problem of accumulation of the low-avidity population managing to isolate high-avidity T lymphocytes such as the clone hTERT20.10 (as in described in the letter by Ugel Stefano http://www.bloodjournal.org/content/115/7/1374.e-letters#re-evidence-for-presentation-of-htert865-873-on-tumor-cells).

Identification of the $\alpha$ and $\beta$ chains of the TCR specific for the peptide hTERT865-873

The RNA was extracted according to the instructions of the TRI Reagent (Life Technologies). In short, the cell pellet was resuspended in 1 mL of TRI reagent and, after 5 minutes incubation at room temperature (RT), 200 $\mu$L of chloroform were added. After transferring the samples in 1.5 mL Phase Lock Gel tubes (5 PRIME), they were centrifuged at maximum speed for 10 minutes at 4 °C and the aqueous phase was transferred into a clean tube. The precipitation of the RNA was performed by addition of isopropanol and, after appropriate centrifugation, ethanol 75%. After a further centrifugation, the supernatant was then removed, the RNA pellet allowed to air dry for 5 minutes, then resuspended in 30 $\mu$L of pyrogen-free water. The final solution was quantified by means of a NanoDrop spectrophotometer (Thermo Scientific).

The reverse transcription to cDNA was carried out by exactly following the instructions of the High Capacity Reverse Transcription Kit (Applied Biosystems). The RNA was diluted to 100 ng/mL with RNAse-free water. For' the reverse transcription reaction, 1 μg of RNA was added to a solution containing: 2 μL buffer 10X, 0.8 μL 25X dNTP Mix (100 mM), 2 μL 10X RT Random Primers, 1 μL Reverse Transcriptase, 50 U/μL, 1 μL RNase Inhibitor (all reagents contained in the kit) and RNAase-free water, for a final volume of 20 μL. The reaction steps were the following: 10 min at 25 °C, 120 min at 37 °C, 5 min at 85 °C, 4 °C until removal.

The quality of the cDNA thus synthesised was assessed by PCR amplification of β-actin murine gene using the primers:

> Forward (Fw) 5'-ctaaggccaaccgtgaaaag-3'
> Reverse (Rev) 5'-ggagagcatagccctcgtag-3'

The reaction was carried out by mixing the reagents in the following quantities: 5.5 μL non-pyrogenic water, 7.5 μL AmpliTaq Gold 360 Master Mix (Applied Biosystems), 0.5 μL Fw primer 10 μM and 0.5 μL Fw primer 10 μM and 0.5 μL cDNA. The reaction cycles were carried out with a Veriti 96-well thermal cycler (Applied Biosystems): 96 °C 5 min, (94 °C 30 sec, 60 °C 45 sec, 72 °C 10 min) x 30 cycles and 72 °C 10 min. This reaction produced a band of the correct molecular weight of 180 bp.

The CDR3 region of the TCRα was identified by PCR using a primer panel specific for the TCR AV subfamilies, listed in Table A of Figure 4.

The first and the second PCR were conducted in 20 μL of volume containing 7.5 μL AmpliTaq Gold 360 Master Mix (Applied Biosystems), 0.5 μL primer and Fw primer Rev, both at a concentration of 10 μM (final concentration of the primers 0.3 μM) and 2 μL of a 1/10 dilution of the cDNA obtained by RT. Both PCRs were performed for 35 cycles of 94 °C 30 sec, 60 °C 45 sec and 72 °C for 10 min followed by final elongation of 10 min at 72 °C. The PCR products (4 μL) were analysed by run on 2% agarose gel. For the first PCR, alternative mixes of 4 Fw primers were used, each plus a constant Rev primer. The only mix that allowed the amplification of a fragment was mix A. The second PCR series was conducted using as Fw primer, alternatively, MAV1, MAV2, MAV5 and MAV12 paired with a constant Rev primer (Table A, Figure 4. The primers that allowed the amplification of a fragment of the expected molecular weight were MAV1 and MAV5, suggesting that the TCR hTERT clone uses a member of one of two AV subfamilies. The two PCR fragments were analysed by direct sequencing, using the Rev constant primer.

The sequence data was analysed by alignment on the IMGT website (http://www.imgt.org) revealing that the clone expressed two TCRα chains: TRAV7D/TRAJ23*01/TRAC e TRAV3/TRAJ11*01/TRAC, but only the sequence of the TCR TRAV3/TRAJ11*01/TRAC is in-frame coherent with the synthesis of a functional TCRα protein chain.

On the basis of the sequence data, a new pair of primers was designed in order to amplify a cDNA fragment containing the entire sequence encoding the chain TCRα TRAV3/TRAJ11*01/TRAC. The PCR reaction was carried out in a volume of 20 μL containing 7.5 μL AmpliTaq Gold 360 Master Mix (Applied Biosystems), 0.5 μL Fw primer, 0.5 μM Rev primer, both at a concentration of 10 μM and 1 L of a 1/10 dilution of the cDNA obtained by RT. The PCR conditions were the same as those described previously in this paragraph, with the only variant of 40 cycles instead of 35. The expected PCR product (841 bp) was assessed by running on 2% agarose gel and sequenced according to the previously described method.

The TCRβ chain was identified using the same strategy described for the TCRα chain. The primers used for the amplification of the CDR3 fragment are listed in Table B, Figure 4 and were used in PCR reactions according to the conditions reported for the corresponding reactions of the TCRα chain. DNA fragments of expected molecular weight were amplified in the presence of the primer D mix and in second PCR of the primer pair MVb3 and CbetaAS (Table B Figure 4). The sequencing of the amplified segment revealed that the TCRβ chain consists of TRBV26/TRBJ2-7*01/TRBD2*01. Furthermore, the DNA sequence is inserted in-frame in specific plasmids (see Fig. 7 and Fig. 8) and appears to encode a functional chain. The complete TCRβ sequence was identified and amplified (same PCR conditions) using the pair of primers shown in Table B, Figure 4, of which the Rev primer appears on the constant portion C2. In conclusion, TCRβ consists of TRBV26*01/TRBJ2-7*01/TRBD2*01/TRBC2*03.

Cloning and sequencing of the sequences of TCRα and TCRβ chains in pcDNA3.1

The complete cDNA sequences encoding TCRα and TCRβ were ligated in the vector TA pCR2.1-Topo cloning (Invitrogen) according to the manufacturer's instructions. In particular, the cDNA sequence of TCRα was amplified as described in the previous paragraph; the TCRβ sequence was amplified with the primers:

> Fw 5'-ttttcggaattctaagccaccat-3'
> Rev 5'-gcttaaacgtttgtctcaggaattt-3'

and respectively containing the restriction sites EcoRI and Pmel for subsequent clonings. In the latter case, the variants of the PCR reaction were a coupling temperature of 58 °C and 40 reaction cycles. The PCR products for TCRα and TCRβ were run on 2% agarose gel and purified using the QIAquick Gel Extraction Kit (Qiagen). For the ligation reaction, the inserts were mixed in a 3:1 ratio with respect to the vector, in the presence of 1 μL of T4 ligase enzyme. The reaction mixture was incubated for 18 hours at 4 °C. 5 μL of the ligation product was transformed into competent *Escherichia coli* TOP10 bacteria (Invitrogen) using thermal shock. The bacterial clones were checked for the presence of the TCRα or TCRβ chain by enzymatic digestion with EcoRI (Neb) of the plasmid DNA obtained using the QIAamp DNA Mini kit (Qiagen). The orientation of the sequence within the vector was determined by digestion with the enzyme PstI (Neb) for TCRα and sequencing for TCRβ. The sequences encoding TCRα and TCRβ were subsequently sub-cloned in the pcDNA3.1 vector using the enzyme EcoRI (Fig. 6).

For all sequencing reactions, 10 μL of DNA were purified by adding 2 μL of ExoSAP-IT® (USB Corporation) and incubating at 37 °C for 30 min plus heat inactivation at 80 °C for 15 min. The subsequent sequencing reaction was carried out in 10 μL of reaction according to the instructions of the BigDye Terminator v3.1 Cycle Sequencing Kit (Applied Biosystems) using BigDye® Terminator Ready Reaction Mix v3.1 1.0 μL, primer (3.2 pmol/pL) 1.0 μL, 1.5 μL buffer, DNA 2.0 μL. The thermal cycler conditions are inactivation at 96 °C for 1 min, followed by 35 cycles of 96 °C for 10 sec, 50 °C for 5 sec and 60 °C for 4 min. The reaction product was purified with GE purification columns and analysed in sequencer ABI 3130xlGA (Applied Biosystems).

### Optimisation of the construct for expression in retroviral vector

Subsequently, a construct (TCR optimised) was created containing: the optimised sequences of the α and β chains of the TCR specific for the hTERT$_{865-873}$ peptide; two cleavage sites P2A and T2A to allow a balanced expression of the two engineered α and β chains; the sequence encoding the truncated form of the CD34 molecule to allow enrichment and identification of the transfected cells (Fig. 7). The construct was then inserted into a retroviral vector (kindly provided by Prof. M. I. Nishimura-Chicago, USA) in order to efficiently transduce human PBMCs (pg1SAMEN-CMVSRa + telomerase optimised TCR - Fig. 8). In addition, Prof. Nishimura also provided the aforesaid vector coding for the α and β chains of the TCR specific for the HCV1406-1415 peptide, which was used as a control.

### Retroviral production and infection of human peripheral mononuclear cells

For the production of the retroviral recombinant proteins, the method of precipitation with calcium chloride was used. Briefly, the plasmid vectors coding for key proteins for virus assembly were purchased from the Takara company. Calcium chloride $CaCl_2$ (2 mM) was added to the solution containing the pGp vector, the pE-eco vector and the vector encoding the TCR (10 μg). Subsequently the buffer HBS 2X (274 mM $NaCl_2$, 10 mM KCl, 1.5 mM $Na_2HPO_4$, 12 mM dextrose, 42 mM Hepes, pH 7.1) was added under stirring. The transfection mix was then added dropwise to the culture plate of 293T cells and the medium containing the viral particles was harvested after 48 hours, centrifuged and filtered in filters with 0.45 μm pores to remove cells and debris and concentrated by ultracentrifugation at 50,000 g for 140 minutes at room temperature.

The human PBMCs were kept in culture in AIM-V medium supplemented with human serum (5%), anti-hCD3 (30 ng/ml), hIL2 (300 IU/ml) and hIL15 (100 mg/ml), so to stimulate the proliferation of the T lymphocyte component. The infection was carried out by incubating $2 \times 10^6$ cells/mL in culture medium and adding equal volumes of medium supplemented with the virus encoding for the TCR specific for hTERT$_{865-873}$ or for HCV1406-1415 (as control) at 20 MOI. After 24 hours of incubation at 37 °C and 5% $CO_2$, the medium was replaced and the cells expanded. In order to verify a successful gene transfer, a flow cytometry control was performed of the clonal populations with high expression of the TCR of our interest. The cells were then washed in saline solution (PBS 1X) and the TCR chain of our interest was marked for 30 minutes at 4 °C with anti-hCD8, anti-hCD34 and anti-mVβ3 antibodies (Fig. 9A).

After checking for the occurrence of the infection and the presence of the correct TCR, the cells were enriched by immunomagnetic method using the Sorting hCD34 MicroBead Kit (Miltenyi Biotec). Briefly, the cells are marked in MACS Buffer with magnetic beads conjugated with the monoclonal antibody specific for hCD34. After 30 minutes incubation at 4 °C, the excess beads were eliminated and the sample was made to flow in a column previously balanced for magnetic separation. The fraction positive for CD34 is retained on the column and eluted successively by moving away from the magnetic field.

### Validation of the specificity of the TCR for the hTERT865-873 peptide and its effectiveness in the recognition of immortalised cell lines expressing telomerase

The specificity for the hTERT$_{865-873}$ peptide was assessed by incubation of the transduced T lymphocytes and the control PBMCs, with the tetramer specific for hTERT$_{865-873}$ or the control tetramer (Fig. 9B), as per the manufacturer's instructions (ProImmune).

The levels of expression and functional activity of the telomerase were evaluated in different human lines of immortalised malignant neoplasias: DG75 (Burkitt's lymphoma), JVM13 (chronic B - B CLL lymphocytic leukaemia), SK23mel (melanoma), PT45 (pancreatic adenocarcinoma), MDAMB231 (breast cancer), all restricted to HLA-A2+ and Raji, Burkitt's lymphoma restricted for HLA-A2-. All the lines express high levels of telomerase (data not reported) and were recognised by the T lymphocytes specific for hTERT$_{865-873}$ after co-culture *in vitro* in HLA-A2-restricted mode. The recognition was assessed in terms of release of $^{51}$Cr and IFNγ (Figures 10A and 10B). The first was carried out as previously described, while as regards the release assay of IFNy, the target cells were put in culture with the effector cells at a ratio of 3:1 in a 96-well plate in duplicate for 24 hours. The supernatant was then transferred to the plate for ELISA, as already explained. This data confirmed the physiological presence of the complex HLA/hTERT$_{865-873}$ peptide in the membrane of tumour cells with different histology and the ability of the isolated sequence to encode a high-avidity functional TCR able to recognise this complex.

Specificity and efficacy of the engineered clone in vivo in the human chronic lymphocytic leukaemia model JVM13

Ten immunodeficient NOG mice were injected with a JVM13 clone, expressing luciferase, intravenously ($10^6$ cells) and were subsequently treated with two adoptive transfers, weekly, of $5\times10^6$ Transgenic T lymphocytes specific for the hTERT$_{865-873}$ peptide or for the control peptide, both followed by intraperitoneal administration of IL-2 (180,000 IU/mouse). The expansion of the tumour cells was monitored weekly by bioluminescence (Fig. 11A). Measurements were made before the start of treatment and then weekly until the time of sacrifice. The animals were anaesthetised with Isoflurane and dorsal and frontal images were acquired for each of them 10 minutes before and after intraperitoneal injection of luciferin (150 mg/kg - PerkinElmer), to be able to discriminate between the specific emission and the baseline. The acquisition parameters were: exposure time = 5 minutes, field of view = 23x23 cm, binninig B = 8 and f/stop = 2. The signals were then quantified by plotting the region of interest (ROI) on the total animal. The images were then acquired with the IVIS Spectrum Imaging System (PerkinElmer). When the specific treatment induced a significant decrease in radiance signals, the mice were sacrificed and the spread of leukaemia cells to the various organs was assessed by flow cytometry and bioluminescence (Fig. 11B and 11C). As regards the flow cytometry, the organs (spleen, bone marrow, liver and lungs) were processed mechanically and the cell suspension was marked with anti-hCD19, anti-hCD45 and anti-mCD45 antibodies to make the marking more precise, all at 4 °C for 30 minutes. The same organs, before processing were put in contact with luciferin in a dark 24-well plate and the bioluminescence images were detected as previously described.

Finally, the therapeutic efficacy of the engineered CTL was also confirmed *in vivo* against solid tumours of different histological origin. The tumour cell lines previously tested *in vitro* were inoculated ($10^6$/mouse) subcutaneously in immunodeficient NOG mice. When the tumour mass reached 125 mm$^3$, the animals were treated with two adoptive transfer of CTL specific for the hTERT$_{865-873}$ peptide or for the control peptide, always followed by administration of IL-2 (180,000 IU/mouse). The adoptive therapy specific for telomerase has shown, even in the case of solid tumours, to be able to significantly control tumour growth, compared to the control, but more important, to be also able to improve overall survival (Fig. 12).

Haematological neoplasias other than chronic lymphocytic leukaemia

An ELISA assay was performed for the release of IFN-γ after 24 hours of co-culture. The assay showed that the lymphocytes specific for hTERT865-873 specifically recognised the T2 line pulsed with the hTERT865-873 peptide but not the same line pulsed with the control peptide. In addition, they were able to specifically recognise both the immortalised line of acute lymphoblastic leukaemia ALL-CM and the acute myeloid leukaemia cell line THP1, both positive for HLA-A2.

This shows that the lymphocytes specific for hTERT865-873 are able to recognise *in vitro* cells derived from acute lymphoblastic leukaemia and acute myeloid leukaemia.

In an *in vivo* assay, the ALL-CM line was inoculated intravenously into NOG mice ($5\times10^6$ cells/mouse) and when the human B cells in circulation reached 5%, the animals were treated with four adoptive transfer of CTL specific for the hTERT$_{865-873}$ peptide or for the control peptide, always followed by administration of IL-2 (180000 IU/mouse). The animal was sacrificed when there are more than 65% of human B cells in the circulation. At sacrifice, spleen and bone marrow were tested in IHC to assess the presence of leukemic cells in spleen (A) and bone marrow (B). Figure 14 shows the assay results.

**Claims**

1. Isolated genetic sequence corresponding to the sequence SEQ. ID. No. 1 which expresses the α chain and the isolated genetic sequence corresponding to the sequence SEQ. ID. No. 2 which expresses the β chain of the T cell receptor (TCR) specific to the complex HLA-A02 and the peptide of telomerase hTERT$_{865-873}$.

2. A construct comprising the genetic sequences of claim 1.

3. The construct according to the preceding claim, which is represented by a plasmid.

4. A vector comprising the construct according to claim 2.

5. The vector according to the preceding claim, represented by a retroviral or lentiviral vector.

6. An isolated cell transduced with the vector according to claim 5.

7. An isolated lymphocyte T cell transduced with the sequence according to claim 1.

8. The isolated lymphocyte T cell according to claim 6 or 7, which is an isolated cell of a mammal.

9. The isolated lymphocyte T cell according to claim 8, which is an isolated human cell.

10. A cell isolated lymphocyte T according to any one of claims 6 to 9 for use as a medicament.

11. The isolated lymphocyte T cell according to the preceding claim, for use as a medicament in the diagnosis or in the treatment of neoplastic diseases.

12. The isolated lymphocyte T cell for use according to the preceding claim, wherein the therapy comprises the technique of adoptive transfer of said cells.

13. The isolated lymphocyte T cell according to any one of claims 10 to 12 for use as medicament for the diagnosis or treatment of neoplastic diseases, wherein said neoplastic diseases comprise solid tumours and haematological neoplasias.

14. The isolated lymphocyte T cell for use according to the preceding claim, wherein said neoplastic diseases include malignant neoplasias such as: Burkitt's lymphoma, chronic lymphocytic leukaemia, melanoma, pancreatic adenocarcinoma, breast cancer, colon cancer, acute lymphoblastic leukaemia and acute myeloid leukaemia.

15. Use of one or both of the sequences according to claim 1 for the generation of isolated lymphocyte T cells specific for the peptide hTERT865-873 **characterised by** the amino acid sequence corresponding to SEQ. ID. no. 7.

16. Use of a vector according to claim 4 for the generation of isolated lymphocyte T cells specific for the peptide hTERT6-873 **characterised by** the amino acid sequence corresponding to SEQ. ID. no. 7.

17. Method for the generation of a vector for the transfection of isolated lymphocyte T cells comprising the use of one or both sequences having a sequence corresponding to SEQ. ID. no. 1 or 2.

18. Method for the generation of a vector for the transfection of isolated lymphocyte T cells comprising the use of a construct comprising one or both sequences having a sequence corresponding to SEQ. ID. no. 1 or 2.

19. Method according to claim 18, wherein said vector is a retroviral vector.

20. Method according to claim 18 or 19, wherein said vector is a retrovirus or a lentivirus.

**Patentansprüche**

1. Isolierte genetische Sequenz entsprechend der Sequenz SEQ. ID. Nr. 1, welche die α-Kette ausdrückt, und die isolierte genetische Sequenz entsprechend der Sequenz SEQ. ID. Nr. 2, welche die β-Kette des T-Zellen-Rezeptors (TCR) ausdrückt, welcher für den Komplex HLA-A02 und das Peptid einer Telomerase hTERT$_{865-873}$ spezifisch ist.

2. Konstrukt, welches die genetischen Sequenzen nach Anspruch 1 umfasst.

3. Konstrukt nach dem vorhergehenden Anspruch, welches durch ein Plasmid repräsentiert ist.

4. Vektor, welcher das Konstrukt nach Anspruch 2 umfasst.

5. Vektor nach dem vorhergehenden Anspruch, repräsentiert durch einen retroviralen oder lentiviralen Vektor.

6. Isolierte Zelle, welche mit dem Vektor nach Anspruch 5 transduziert ist.

7. Isolierte Lymphozyten-T-Zelle, welche mit der Sequenz nach Anspruch 1 transduziert ist.

8. Isolierte Lymphozyten-T-Zelle nach Anspruch 6 oder 7, welche eine isolierte Zelle eines Säugetiers ist.

9. Isolierte Lymphozyten-T-Zelle nach Anspruch 8, welche eine isolierte menschliche Zelle ist.

10. Isolierte Lymphozyten-T-Zelle nach einem der Ansprüche 6 bis 9 zur Verwendung als ein Medikament.

11. Isolierte Lymphozyten-T-Zelle nach dem vorhergehenden Anspruch zur Verwendung als ein Medikament bei der Diagnose oder bei der Behandlung neoplastischer Erkrankungen.

12. Isolierte Lymphozyten-T-Zelle zur Verwendung nach dem vorhergehenden Anspruch, wobei die Therapie die Technik eines adoptiven Transfers der Zellen umfasst.

13. Isolierte Lymphozyten-T-Zelle nach einem der Ansprüche 10 bis 12 zur Verwendung als Medikament zur Diagnose oder Behandlung neoplastischer Erkrankungen, wobei die neoplastischen Erkrankungen solide Tumore und hämatologische Neoplasien umfassen.

14. Isolierte Lymphozyten-T-Zelle zur Verwendung nach dem vorhergehenden Anspruch, wobei die neoplastischen Erkrankungen bösartige Neoplasien wie die Folgenden umfassen: Burkitt-Lymphom, chronische lymphatische Leukämie, Melanom, Pankreas-Adenokarzinom, Brustkrebs, Dickdarmkrebs, akute lymphatische Leukämie und akute myeloische Leukämie.

15. Verwendung einer oder beider Sequenzen nach Anspruch 1 zur Erzeugung isolierter Lymphozyten-T-Zellen, welche für das Peptid hTERT865-873 spezifisch sind, **gekennzeichnet durch** die Aminosäuresequenz entsprechend SEQ. ID. Nr. 7.

16. Verwendung eines Vektors nach Anspruch 4 zur Erzeugung isolierter Lymphozyten-T-Zellen, welche für das Peptid hTERT6-873 spezifisch sind, **gekennzeichnet durch** die Aminosäuresequenz entsprechend SEQ. ID. Nr. 7.

17. Verfahren zur Erzeugung eines Vektors zur Transfektion isolierter Lymphozyten-T-Zellen, umfassend die Verwendung einer oder beider Sequenzen mit einer Sequenz entsprechend SEQ. ID. Nr. 1 oder 2.

18. Verfahren zur Erzeugung eines Vektors zur Transfektion isolierter Lymphozyten-T-Zellen, umfassend die Verwendung eines Konstrukts, welches eine oder beide Sequenzen mit einer Sequenz entsprechend SEQ. ID. Nr. 1 oder 2 umfasst.

19. Verfahren nach Anspruch 18, wobei der Vektor ein retroviraler Vektor ist.

20. Verfahren nach Anspruch 18 oder 19, wobei der Vektor ein Retrovirus oder ein Lentivirus ist.

**Revendications**

1. Séquence génétique isolée correspondant à la séquence SEQ. ID. No. 1 qui exprime la chaîne $\alpha$ et séquence génétique isolée correspondant à la séquence SEQ. ID. No. 2 qui exprime la chaîne $\beta$ du récepteur des cellules T (TCR) spécifique du complexe HLA-A02 et du peptide de télomérase hTERT$_{865-873}$.

2. Construction comprenant les séquences génétiques de la revendication 1.

3. Construction selon la revendication précédente, qui est représentée par un plasmide.

4. Vecteur comprenant la construction selon la revendication 2.

5. Vecteur selon la revendication précédente, représenté par un vecteur rétroviral ou lentiviral.

6. Cellule isolée transduite avec le vecteur selon la revendication 5.

7. Cellule T lymphocytaire isolée transduite avec la séquence selon la revendication 1.

8. Cellule T lymphocytaire isolée selon la revendication 6 ou 7, qui est une cellule isolée d'un mammifère.

9. Cellule T lymphocytaire isolée selon la revendication 8, qui est une cellule humaine isolée.

10. Lymphocyte T isolé de cellule selon l'une quelconque des revendications 6 à 9 pour une utilisation comme médicament.

11. Cellule T lymphocytaire isolée selon la revendication précédente, pour une utilisation comme médicament dans le diagnostic ou le traitement de maladies néoplasiques.

12. Cellule T lymphocytaire isolée pour une utilisation selon la revendication précédente, dans laquelle la thérapie comprend la technique de transfert adoptif desdites cellules.

13. Cellule T lymphocytaire isolée selon l'une quelconque des revendications 10 à 12 pour une utilisation comme médicament pour le diagnostic ou le traitement de maladies néoplasiques, dans laquelle lesdites maladies néoplasiques comprennent les tumeurs solides et les néoplasies hématologiques.

14. Cellule T lymphocytaire isolée pour une utilisation selon la revendication précédente, dans laquelle lesdites maladies néoplasiques comprennent les néoplasies malignes telles que : le lymphome de Burkitt, la leucémie lymphoïde chronique, le mélanome, l'adénocarcinome pancréatique, le cancer du sein, le cancer du côlon, la leucémie lymphoblastique aiguë et la leucémie myéloïde aiguë.

15. Utilisation de l'une ou des deux séquences selon la revendication 1 pour la génération de cellules T lymphocytaires isolées spécifiques du peptide hTERT865-873 **caractérisé par** la séquence d'acides aminés correspondant à SEQ. ID. no. 7.

16. Utilisation d'un vecteur selon la revendication 4 pour la génération de cellules T lymphocytaires isolées spécifiques du peptide hTERT6-873 **caractérisé par** la séquence d'acides aminés correspondant à SEQ. ID. no. 7.

17. Procédé de génération d'un vecteur pour la transfection de cellules T lymphocytaires isolées comprenant l'utilisation d'une ou des deux séquences ayant une séquence correspondant à SEQ. ID. no. 1 ou 2.

18. Procédé de génération d'un vecteur pour la transfection de cellules T lymphocytaires isolées comprenant l'utilisation d'une construction comprenant une ou les deux séquences ayant une séquence correspondant à SEQ. ID. no. 1 ou 2.

19. Procédé selon la revendication 18, dans lequel ledit vecteur est un vecteur rétroviral.

20. Procédé selon la revendication 18 ou 19, dans lequel ledit vecteur est un rétrovirus ou un lentivirus.

Fig. 1

A

B

Fig. 2

Fig. 3

| Cloning (cells/wells) | Number of clones | Cloning efficiency (%) | Low avidity clones | High avidity clones |
|---|---|---|---|---|
| 100 | 14 | 3.64 | 12 | 2 |
| 30 | 9 | 2.34 | 8 | 1 |
| 3 | 2 | 0.56 | 2 | 0 |

Fig. 4

*Oligonucleotides specific for TRAV and TRAC*

| IMGT sub-group [1] | Name | Sequence 5'-3' | PCR MIX |
|---|---|---|---|
| TRAV7 | MAV1 | cccagcccagtgactccgctctc | Mix A |
| TRAV14 | MAV2 | ctcagcctggagactcagccacc | Mix A |
| TRAV3 | MAV5 | gcccatcctggggactcagccg | Mix A |
| TRAV2 | MAV12 | cctgggtttccctgagagatgctg | Mix A |
| TRAV9 | MAV3 | gagttcagcaagagtaactc | Mix B |
| TRAV15 | MAV7 | ttacaaccagacgattcggg | Mix B |
| TRAV13 | MAV10 | cctcctcccagatcacag | Mix B |
| TRAV10 | MAV15 | ccacacagcctgaagattca | Mix B |
| TRAV21 | MAV6 | tcgatcctgcctcatgtcagc | Mix C |
| TRAV4 | MAV11 | ccagtcggaggactcaggc | Mix C |
| TRAV11 | MAV14 | cacagcacgctgcacatcacag | Mix C |
| TRAV1 | MAV19 | ctgacagagctccagatcaaagac | Mix C |
| TRAV20 | MAV16 | gcacgaacagaagattcagcc | Mix D |
| TRAV16 | MAV17 | gccacacagattgaggactc | Mix D |
| TRAV8 | MAV18 | tgtgaagacaccgctgtgtac | Mix D |
| TRAV19 | MAV20 | gcctgaagacacagcagtg | Mix D |
| TRAV6 | MAV4 | cacttgcagaaagcctcatgtc | Mix E |
| TRAV12 | MAV8 | tccttccatctgcagaagtcc | Mix E |
| TRAV17 | MAV9 | cctgaagaaatccccagccc | Mix E |
| TRAV5 | MAV13 | ccctgcacatcacagacacc | Mix E |
|  | MACoantiAS | caggacagcaccctctgcctg |  |

A

*Oligonucleotides specific for TRBV and TRBC*

| IMGT sub-group [1] | Name | Sequence 5'-3' | PCR MIX |
|---|---|---|---|
| TRBV12-2 | MVb5.1 | cct tgg agc tag agg act ctg ccg | Mix A |
| TRBV13-2 | MVb8.2 | gct acc ccc tct cag aca tca gtg | Mix A |
| TRBV16 | MVb11 | tga aga tcc aga gca gcg ggc ccc | Mix A |
| TRBV31 | MVb14 | gca cgg aga agc tgc ttc tca gcc | Mix A |
| TRBV19 | MVb6 | gcc cag aag aac gag atg gcc gtt | Mix B |
| TRBV15 | MVb12 | cca ctc tga aga ttc aac cta cag aac cc | Mix B |
| TRBV3 | MVb16 | ctc tga aaa tcc aac cca cag cac tgg | Mix B |
| TRBV30 | MVb18 | gca agg cct gga gac agc agt atc | Mix B |
| TRBV2 | MVb4 | cta aag cct gat gac tcg gcc aca | Mix C |
| TRBV12-1 | MVb5.2 | cct tgg aac tgg agg act ctg cta | Mix C |
| TRBV29 | MVb7 | gga ttc tgc taa aac aaa cca gac atc tgt | Mix C |
| TRBV17 | MVb9 | ctc tct cta cat tgg ctc tgc agg | Mix C |
| TRBV4 | MVb10 | ctt cga atc aag tct gta gag ccg g | Mix C |
| TRBV1 | MVb2 | atg agc cag ggc aga acc ttg tac | Mix D |
| TRBV26 | MVb3 | gaa att cag tcc tct gag gca gga | Mix D |
| TRBV13-1 | MVb8.3 | ggc ttc tcc ctc tca gac atc tt | Mix D |
| TRBV14 | MVb13 | caa gat cca gtc tgc aaa gca ggg | Mix D |
| TRBV5 | MVb1 | cag aca gct cca agc tac ttt tac | Mix E |
| TRBV13-3 | MVb8.1 | gct tcc ctt tct cag aca gct gta | Mix E |
| TRBV20 | MVb15 | gca tat ctt gaa gac aga ggc | Mix E |
| TRBV24 | MVb17 | tct gaa gaa gac gac tca gca ctg | Mix E |
|  | Cbeta AS | agg aga cct tgg gtg gag tca |  |

B

Fig. 5

<table>
<tr><th colspan="4">A</th></tr>
<tr><td></td><th colspan="3">TCR – chain 5α</th></tr>
<tr><th>Restriction HLA</th><th>AV</th><th>CDR 5α</th><th>AJ</th></tr>
<tr><td>A2-02*01</td><td>TRAV3D-3*02<br><br>or<br><br>TRAV3N-3*01</td><td>CAVRHDSGYNKLTF</td><td>TRAJ11*01</td></tr>
<tr><td></td><td></td><td></td><td></td></tr>
<tr><td></td><td></td><th>TCR – chain 3β</th><td></td></tr>
<tr><th>Restriction HLA</th><th>BV</th><th>CDR 3 β</th><th>BJ</th></tr>
<tr><td>A2-02*01</td><td>TRBV26*01</td><td>CASSLSKGGFYEQYF</td><td>TRBJ2-7*01</td></tr>
</table>

<table>
<tr><th colspan="2">B</th></tr>
<tr><th>Sequenza</th><th>CATENA TCR</th></tr>
<tr><td>ATGAAGACAGTGACTGGACCTTTGTTCCTGTGCTTCTGGCTGCAGCTGAACTGTGTGAGCAGAGG<br>CGAGCAGGTGGAGCAGCGCCCTCCTCACCTGAGTGTCCGGGAGGGAGACAGTGCCGTTATCATCT<br>GCACCTACACAGACCCTAACAGTTATTACTTCTTCTGGTACAAGCAAGAGCCGGGGGCAGGTCTT<br>CAGTTGCTTATGAAGGTTTTCTCAAGTACGGAAATAAACGAAGGACAAGGATTCACTGTCCTACT<br>GAACAAGAAAGACAAACAACTCTCTCTGAACCTCACAGCTGCCCATCCTGGGGACTCAGCCGTGT<br>ACTTCTGCGCAGTCAGGCATGACTCGGGATACAACAAACTCACTTTTGGAAAGGGCACGGTGCTT<br>CTAGTCTCTCCAGACATCCAGAACCCAGAACCTGCTGTGTACCAGTTAAAAGATCCTCGGTCTCA<br>GGACAGCACCCTCTGCCTGTTCACCGACTTTGACTCCCAAATCAATGTGCCGAAAACCATGGAAT<br>CTGGAACGTTCATCACTGACAAAACTGTGCTGGACATGAAAGCTATGGATTCCAAGAGCAATGGG<br>GCCATTGCCTGGAGCAACCAGACAAGCTTCACCTGCCAAGATATCTTCAAAGAGACCAACGCCAC<br>CTACCCCAGTTCAGACGTTCCCTGTGATGCCACGTTGACTGAGAAAAGCTTTGAAACAGATATGA<br>ACCTAAACTTTCAAAACCTGTCAGTTATGGGACTCCGAATCCTCCTGCTGAAAGTAGCCGGATTT<br>AACCTGCTCATGACGCTGAGGCTGTGGTCCAGTTGA</td><td>α5</td></tr>
<tr><td>ATGGCTACAAGGCTCCtCTGTTACACAGTACTTTGTCTCCtgggtgcaagAATTTTGAATTCaAA<br>AGTCATTCAGACTCCAAGATATCTGGTGAAAGGGCAAGGACAAAAAGCAAAGATGAGGTGTATCC<br>CTGAAAAGGGACATCCAGTtGtatTCTGGTATCAACAAAATAAGAACAATGAGTTTAAATTTTTG<br>ATTAACTTTCAGAATCAAGAAGTTCTTCAGCAAATAGACATGACTGAAAAACGATTCTCTGCTGA<br>GTGTCCTTCAAACTCACCTTGCAGCCTAGAAATTCAGTCCTCTGAGGCAGGAGACTCAGCACTGT<br>ACCTCTGTGCCAGCAGTCTGTCGAAGGGGGGGTTCTATAACAGTACTTCGGTCCCGGCACCAGG<br>CTCACGGTTTTAGAGGATCTGAGAAATGTGACTCCACCCAAGGTCTCCTTGTTTGAGCCATCAAA<br>AGCAGAGATTGCAAACAAACAAAAGGCTACCCTCGTGTGCTTGGCCAGGGGCTTCTTCCCTGACC<br>ACGTGGAGCTGAGCTGGTGGGTGAATGGCAAGGAGGTCCACAGTGGGGTCAGCACGGACCCTCAG<br>GCCTACAAGGAGAGCAATTATAGCTACTGCCTGAGCAGCCGCCTGAGGGTCTCTGCTACCTTCTG<br>GCACAATCCTCGAAACCACTTCCGCTGCCAAGTGCAGTTCCATGGGCTTTCAGAGGAGGACAAGT<br>GGCCAGAGGGCTCACCCAAACCTGTCACACAGAACATCAGTGCAGAGGCCTGGGGCCGAGCAGaC<br>TGTGGAATCACTTCAGCATCCTATCATCAGGGGGTTCTGTCTGCAACCATCCTCTATGAGATCCT<br>ACTGGGGAAGGCCACCCTATATGCTGTGCTGGTCAGTGGCCTGGTGCTGATGGCCATGGTCAAGA<br>AAAAAAATTCCTGA</td><td>β3</td></tr>
</table>

Fig. 6

**A**

Fig. 6A

```
gacggatcgg gagatctccc gatcccctat ggtgcactct cagtacaatc tgctctgatg
ccgcatagtt aagccagtat ctgctccctg cttgtgtgtt ggaggtcgct gagtagtgcg
cgagcaaaat ttaagctaca acaaggcaag gcttgaccga caattgcatg aagaatctgc
ttagggttag gcgttttgcg ctgcttgcg atgtacgggc cagatatacg cgttgacatt
gattattgac tagttattaa tagtaatcaa ttacggggtc attagttcat agcccatata
tggagttccg cgttacataa cttacggtaa atggcccgcc tggctgaccg cccaacgacc
cccgcccatt gacgtcaata atgacgtatg ttcccatagt aacgccaata gggactttcc
attgacgtca atgggtggag tatttacggt aaactgccca cttggcagta catcaagtgt
atcatatgcc aagtacgccc cctattgacg tcaatgacgg taaatggccc gcctggcatt
atgcccagta catgacctta tgggactttc ctacttggca gtacatctac gtattagtca
tcgctattac catggtgatg cggttttggc agtacatcaa tgggcgtgga tagcggtttg
actcacgggg atttccaagt ctccacccca ttgacgtcaa tgggagtttg ttttggcacc
aaaatcaacg ggactttcca aatgtcgta acaactccgc cccattgacg caaatgggcg
gtaggcgtgt acggtgggag gtctatataa gcagagctct ctggctaact agagaaccca
ctgcttactg gcttatcgaa attaatacga ctcactatag ggagacccaa gctggctagc
gtttaaactt aagcttggta ccgagctcgg atccactagt aacggccgcc agtgtgctgg
aattcggctt ttttcggaat tctaagccac catgaagacc gtcactggcc ccctgttcct
gtgcttttgg ctgcagctga attgcgtgtc ccgaggcgag caggtggagc agagaccccc
ccacctgtca gtgcgggagg gcgatagcgc cgtcatcatt tgcacataca ctgaccccaa
ctcctactat ttcttttggt ataaacagga acctggggct ggactgcagc tgctgatgaa
ggtgttcagc tccactgaga ttaatgaagg ccaggggttt accgtcctgc tgaacaagaa
agataagcag ctgagtctga atctgaccgc agctcaccca ggggatagcg ccgtgtactt
ctgcgctgtc agacatgaca gcggatataa caaactgacc tttggaaagg cacagtgct
gctggtctct ccagacatcc agaatcctga gccagccgtg taccagctga aggaccccag
gtctcaggat agcaccctgt gcctgttcac cgactttgat tctcagatta acgtgcccaa
aaccatggaa agtggcacct tcatcacaga caagactgtc ctggatatga aagccatgga
ctccaagtct aacggggcaa ttgcctggag caatcagacc tccttacat gccaggatat
ctttaaagag actaatgcaa cctatccttc tagtgacgtg ccatgtgatg ccaccctgac
agagaagtcc ttcgaaaccg acatgaacct gaattttcag aacctgtctg tgatgggcct
gagaatcctg ctgctgaaag tcgccgggtt taatctgctg atgacactgc gactgtggtc
aagctgagac aaacgtttaa gcaagccgaa ttctgcagat atccatcaca ctggcggccg
ctcgagtcta gagggcccgt ttaaacccgc tgatcagcct cgactgtgcc ttctagttgc
cagccatctg ttgtttgccc ctcccccgtg ccttccttga ccctggaagg tgccactccc
actgtccttt cctaataaaa tgaggaaatt gcatcgcatt gtctgagtag gtgtcattct
attctggggg gtggggtggg gcaggacagc aaggggagg attgggaaga caatagcagg
catgctgggg atgcggtggg ctctatggct tctgaggcgg aaagaaccag ctggggctct
aggggtatc cccacgcgcc ctgtagcggc gcattaagcg cggcgggtgt ggtggttacg
cgcagcgtga ccgctacact tgccagcgcc ctagcgcccg ctcctttcgc tttcttccct
tcctttctcg ccacgttcgc cggctttccc cgtcaagctc taaatcgggg gctccctta
gggttccgat ttagtgcttt acggcacctc gaccccaaaa aacttgatta gggtgatggt
```

Fig. 6A

```
tcacgtagtg ggccatcgcc ctgatagacg gttttttcgcc cttttgacgtt ggagtccacg
ttctttaata gtggactctt gttccaaact ggaacaacac tcaaccctat ctcggtctat
tcttttgatt tataagggat tttgccgatt tcggcctatt ggttaaaaaa tgagctgatt
taacaaaaat ttaacgcgaa ttaattctgt ggaatgtgtg tcagttaggg tgtggaaagt
ccccaggctc cccagcaggc agaagtatgc aaagcatgca tctcaattag tcagcaacca
ggtgtggaaa gtccccaggc tccccagcag gcagaagtat gcaaagcatg catctcaatt
agtcagcaac catagtcccg cccctaactc cgcccatccc gcccctaact ccgcccagtt
ccgcccattc tccgccccat ggctgactaa ttttttttat ttatgcagag gccgaggccg
cctctgcctc tgagctattc cagaagtagt gaggaggctt ttttggaggc ctaggctttt
gcaaaaagct cccgggagct tgtatatcca ttttcggatc tgatcaagag acaggatgag
gatcgtttcg catgattgaa caagatggat tgcacgcagg ttctccggcc gcttgggtgg
agaggctatt cggctatgac tgggcacaac agacaatcgg ctgctctgat gccgccgtgt
tccggctgtc agcgcagggg cgcccggttc tttttgtcaa gaccgacctg tccggtgccc
tgaatgaact gcaggacgag gcagcgcggc tatcgtggct ggccacgacg ggcgttcctt
gcgcagctgt gctcgacgtt gtcactgaag cgggaaggga ctggctgcta ttgggcgaag
tgccggggca ggatctcctg tcatctcacc ttgctcctgc cgagaaagta tccatcatgg
ctgatgcaat gcggcggctg catacgcttg atccggctac ctgcccattc gaccaccaag
cgaaacatcg catcgagcga gcacgtactc ggatggaagc cggtcttgtc gatcaggatg
atctggacga agagcatcag gggctcgcgc cagccgaact gttcgccagg ctcaaggcgc
gcatgcccga cggcgaggat ctcgtcgtga cccatggcga tgcctgcttg ccgaatatca
tggtggaaaa tggccgcttt tctggattca tcgactgtgg ccggctgggt gtggcggacc
gctatcagga catagcgttg gctacccgtg atattgctga agagcttggc ggcgaatggg
ctgaccgctt cctcgtgctt tacggtatcg ccgctcccga ttcgcagcgc atcgccttct
atcgccttct tgacgagttc ttctgagcgg gactctgggg ttcgaaatga ccgaccaagc
gacgcccaac ctgccatcac gagatttcga ttccaccgcc gccttctatg aaaggttggg
cttcggaatc gttttccggg acgccggctg gatgatcctc cagcgcgggg atctcatgct
ggagttcttc gcccacccca acttgtttat tgcagcttat aatggttaca aataaagcaa
tagcatcaca aatttcacaa ataaagcatt tttttcactg cattctagtt gtggtttgtc
caaactcatc aatgtatctt atcatgtctg tataccgtcg acctctagct agagcttggc
gtaatcatgg tcatagctgt ttcctgtgtg aaattgttat ccgctcacaa ttccacacaa
catacgagcc ggaagcataa agtgtaaagc ctggggtgcc taatgagtga gctaactcac
attaattgcg ttgcgctcac tgcccgcttt ccagtcggga aacctgtcgt gccagctgca
ttaatgaatc ggccaacgcg cggggagagg cggtttgcgt attgggcgct cttccgcttc
ctcgctcact gactcgctgc gctcggtcgt tcggctgcgg cgagcggtat cagctcactc
aaaggcggta atacggttat ccacagaatc agggGataac gcaggaaaga acatgtgagc
aaaaggccag caaaaggcca ggaaccgtaa aaaggccgcg ttgctggcgt ttttccatag
gctccgcccc cctgacgagc atcacaaaaa tcgacgctca agtcagaggt ggcgaaaccc
gacaggacta taaagatacc aggcgtttcc ccctggaagc tccctcgtgc gctctcctgt
tccgaccctg ccgcttaccg gatacctgtc cgcctttctc ccttcgggaa gcgtggcgct
ttctcatagc tcacgctgta ggtatctcag ttcggtgtag gtcgttcgct ccaagctggg
```

Fig. 6A

```
ctgtgtgcac  gaaccccccg  ttcagcccga  ccgctgcgcc  ttatccggta  actatcgtct
tgagtccaac  ccggtaagac  acgacttatc  gccactggca  gcagccactg  gtaacaggat
tagcagagcg  aggtatgtag  gcggtgctac  agagttcttg  aagtggtggc  ctaactacgg
ctacactaga  agaacagtat  ttggtatctg  cgctctgctg  aagccagtta  ccttcggaaa
aagagttggt  agctcttgat  ccggcaaaca  aaccaccgct  ggtagcggtt  tttttgtttg
caagcagcag  attacgcgca  gaaaaaaagg  atctcaagaa  gatcctttga  tcttttctac
ggggtctgac  gctcagtgga  acgaaaactc  acgttaaggg  attttggtca  tgagattatc
aaaaaggatc  ttcacctaga  tccttttaaa  ttaaaaatga  agttttaaat  caatctaaag
tatatatgag  taaacttggt  ctgacagtta  ccaatgctta  atcagtgagg  cacctatctc
agcgatctgt  ctatttcgtt  catccatagt  tgcctgactc  cccgtcgtgt  agataactac
gatacgggag  ggcttaccat  ctggccccag  tgctgcaatg  ataccgcgag  acccacgctc
accggctcca  gatttatcag  caataaacca  gccagccgga  agggccgagc  gcagaagtgg
tcctgcaact  ttatccgcct  ccatccagtc  tattaattgt  tgccgggaag  ctagagtaag
tagttcgcca  gttaatagtt  tgcgcaacgt  tgttgccatt  gctacaggca  tcgtggtgtc
acgctcgtcg  tttggtatgg  cttcattcag  ctccggttcc  caacgatcaa  ggcgagttac
atgatccccc  atgttgtgca  aaaaagcggt  tagctccttc  ggtcctccga  tcgttgtcag
aagtaagttg  gccgcagtgt  tatcactcat  ggttatggca  gcactgcata  attctcttac
tgtcatgcca  tccgtaagat  gcttttctgt  gactggtgag  tactcaacca  agtcattctg
agaatagtgt  atgcggcgac  cgagttgctc  ttgcccggcg  tcaatacggg  ataataccgc
gccacatagc  agaactttaa  aagtgctcat  cattggaaaa  cgttcttcgg  ggcgaaaact
ctcaaggatc  ttaccgctgt  tgagatccag  ttcgatgtaa  cccactcgtg  cacccaactg
atcttcagca  tcttttactt  tcaccagcgt  ttctgggtga  gcaaaaacag  gaaggcaaaa
tgccgcaaaa  aagggaataa  gggcgacacg  gaaatgttga  atactcatac  tcttcctttt
tcaatattat  tgaagcattt  atcagggtta  ttgtctcatg  agcggataca  tatttgaatg
tatttagaaa  aataaacaaa  tagggggttcc  gcgcacattt  ccccgaaaag  tgccacctga
cgtc
```

Fig. 6

## B

Fig. 6B

```
gacggatcgg gagatctccc gatcccctat ggtgcactct cagtacaatc tgctctgatg
ccgcatagtt aagccagtat ctgctccctg cttgtgtgtt ggaggtcgct gagtagtgcg
cgagcaaaat ttaagctaca acaaggcaag gcttgaccga caattgcatg aagaatctgc
ttagggttag gcgttttgcg ctgcttcgcg atgtacgggc cagatatacg cgttgacatt
gattattgac tagttattaa tagtaatcaa ttacggggtc attagttcat agcccatata
tggagttccg cgttacataa cttacggtaa atggcccgcc tggctgaccg cccaacgacc
cccgcccatt gacgtcaata atgacgtatg ttcccatagt aacgccaata gggactttcc
attgacgtca atgggtggag tatttacggt aaactgccca cttggcagta catcaagtgt
atcatatgcc aagtacgccc cctattgacg tcaatgacgg taaatggccc gcctggcatt
atgcccagta catgacctta tgggactttc ctacttggca gtacatctac gtattagtca
tcgctattac catggtgatg cggttttggc agtacatcaa tgggcgtgga tagcggtttg
actcacgggg atttccaagt ctccacccca ttgacgtcaa tgggagtttg ttttggcacc
aaaatcaacg ggactttcca aaatgtcgta caactccgc cccattgacg caaatgggcg
gtaggcgtgt acggtgggag gtctatataa gcagagctct ctggctaact agagaaccca
ctgcttactg gcttatcgaa attaatacga ctcactatag ggagacccaa gctggctagc
gtttaaactt aagcttggta ccgagctcgg atccactagt aacggccgcc agtgtgctgg
aattcggctt ttttcggaat tctaagccac catggcaact cgactgctgt gctacaccgt
gctgtgcctg ctgggagcac gcattctgaa ctccaaagtg atccagaccc cccgatatct
ggtcaagggc caggggcaga aagccaagat gcgatgcatt cccgagaagg gacaccctgt
ggtcttctgg taccagcaga acaaaaacaa cgagttcaag tttctgatta actttcagaa
tcaggaagtg ctgcagcaga tcgatatgac cgagaagcgg ttctcagccg aatgccccag
taattcacct tgtagcctgg agatccagtc ctctgaagca ggcgactccg ccctgtatct
gtgcgccagt tcactgtcta agggaggctt ctacgagcag tattttggac ctggcacaag
actgactgtg ctggaagacc tgaggaacgt gactccccct aaagtctctc tgtttgagcc
tagtaaggct gaaatcgcaa acaagcagaa ggccaccctg gtgtgcctgg ccagaggctt
ctttcccgat cacgtggagc tgagctggtg ggtcaacggg aaagaagtgc attcaggagt
cagcacagac cctcaagcct acaaggagtc caattactct tattgcctga gctcccggct
gcgggtgagc gccaccttct ggcacaaccc caggaatcat ttccgctgtc aggtccagtt
tcacggactg tctgaggaag ataaatggcc tgagggcagt cccaagcctg tgacccagaa
catttcagct gaggcctggg gaagggctga ctgtggcatc acaagcgcat cctatcatca
gggcgtgctg tccgccacca tcctgtacga gattctgctg ggcaaggcta cactgtatgc
agtgctggtc tctgggctgg tgctgatggc tatggtcaag aaaaagaact cttgagacaa
acgtttaagc aagccgaatt ctgcagatat ccatcacact ggcggccgct cgagtctaga
gggcccgttt aaacccgctg atcagcctcg actgtgcctt ctagttgcca gccatctgtt
gtttgcccct ccccgtgcc ttccttgacc ctggaaggtg ccactcccac tgtcctttcc
taataaaatg aggaaattgc atcgcattgt ctgagtaggt gtcattctat tctggggggt
ggggtggggc aggacagcaa ggggggaggat tgggaagaca atagcaggca tgctggggat
gcggtgggct ctatggcttc tgaggcggaa agaaccagct ggggctctag ggggtatccc
cacgcgccct gtagcggcgc attaagcgcg gcgggtgtgg tggttacgcg cagcgtgacc
gctacacttg ccagcgccct agcgcccgct cctttcgctt tcttcccttc ctttctcgcc
acgttcgccg gctttccccg tcaagctcta aatcggggggc tccctttagg gttccgattt
agtgctttac ggcacctcga ccccaaaaaa cttgattagg gtgatggttc acgtagtggg
ccatcgccct gatagacggt ttttcgccct ttgacgttgg agtccacgtt ctttaatagt
```

Fig. 6B

```
ggactcttgt tccaaactgg aacaacactc aaccctatct cggtctattc ttttgattta
taagggattt tgccgatttc ggcctattgg ttaaaaaatg agctgattta acaaaaattt
aacgcgaatt aattctgtgg aatgtgtgtc agttagggtg tggaaagtcc ccaggctccc
cagcaggcag aagtatgcaa agcatgcatc tcaattagtc agcaaccagg tgtggaaagt
ccccaggctc cccagcaggc agaagtatgc aaagcatgca tctcaattag tcagcaacca
tagtcccgcc cctaactccg cccatcccgc ccctaactcc gcccagttcc gcccattctc
cgccccatgg ctgactaatt ttttttattt atgcagaggc cgaggccgcc tctgcctctg
agctattcca gaagtagtga ggaggctttt ttggaggcct aggcttttgc aaaaagctcc
cgggagcttg tatatccatt ttcggatctg atcaagagac aggatgagga tcgtttcgca
tgattgaaca agatggattg cacgcaggtt ctccggccgc ttgggtggag aggctattcg
gctatgactg ggcacaacag acaatcggct gctctgatgc cgccgtgttc cggctgtcag
cgcaggggcg cccggttctt tttgtcaaga ccgacctgtc cggtgccctg aatgaactgc
aggacgaggc agcgcggcta tcgtggctgg ccacgacggg cgttccttgc gcagctgtgc
tcgacgttgt cactgaagcg ggaagggact ggctgctatt gggcgaagtg ccggggcagg
atctcctgtc atctcacctt gctcctgccg agaaagtatc catcatggct gatgcaatgc
ggcggctgca tacgcttgat ccggctacct gcccattcga ccaccaagcg aaacatcgca
tcgagcgagc acgtactcgg atggaagccg gtcttgtcga tcaggatgat ctggacgaag
agcatcaggg gctcgcgcca gccgaactgt tcgccaggct caaggcgcgc atgcccgacg
gcgaggatct cgtcgtgacc catggcgatg cctgcttgcc gaatatcatg gtggaaaatg
gccgcttttc tggattcatc gactgtggcc ggctgggtgt ggcggaccgc tatcaggaca
tagcgttggc tacccgtgat attgctgaag agcttggcgg cgaatgggct gaccgcttcc
tcgtgcttta cggtatcgcc gctcccgatt cgcagcgcat cgccttctat cgccttcttg
acgagttctt ctgagcggga ctctggggtt cgaaatgacc gaccaagcga cgcccaacct
gccatcacga gatttcgatt ccaccgccgc cttctatgaa aggttgggct tcggaatcgt
tttccgggac gccggctgga tgatcctcca gcgcggggat ctcatgctgg agttcttcgc
ccaccccaac ttgtttattg cagcttataa tggttacaaa taaagcaata gcatcacaaa
tttcacaaat aaagcatttt tttcactgca ttctagttgt ggtttgtcca aactcatcaa
tgtatcttat catgtctgta taccgtcgac ctctagctag agcttggcgt aatcatggtc
atagctgttt cctgtgtgaa attgttatcc gctcacaatt ccacacaaca tacgagccgg
aagcataaag tgtaaagcct ggggtgccta atgagtgagc taactcacat taattgcgtt
gcgctcactg cccgctttcc agtcgggaaa cctgtcgtgc cagctgcatt aatgaatcgg
ccaacgcgcg gggagaggcg gtttgcgtat gggcgctct tccgcttcct cgctcactga
ctcgctgcgc tcggtcgttc ggctgcggcg agcggtatca gctcactcaa aggcggtaat
acggttatcc acagaatcag gggataacgc aggaaagaac atgtgagcaa aaggccagca
aaaggccagg aaccgtaaaa aggccgcgtt gctggcgttt ttccataggc tccgcccccc
tgacgagcat cacaaaaatc gacgctcaag tcagaggtgg cgaaacccga caggactata
aagataccag gcgtttcccc ctggaagctc cctcgtgcgc tctcctgttc cgaccctgcc
gcttaccgga tacctgtccg cctttctccc ttcgggaagc gtggcgcttt ctcatagctc
acgctgtagg tatctcagtt cggtgtaggt cgttcgctcc aagctgggct gtgtgcacga
accccccgtt cagcccgacc gctgcgcctt atccggtaac tatcgtcttg agtccaaccc
ggtaagacac gacttatcgc cactggcagc agccactggt aacaggatta gcagagcgag
gtatgtaggc ggtgctacag agttcttgaa gtggtggcct aactacggct acactagaag
aacagtattt ggtatctgcg ctctgctgaa gccagttacc ttcggaaaaa gagttggtag
ctcttgatcc ggcaaacaaa ccaccgctgg tagcggtttt tttgtttgca agcagcagat
```

Fig. 6B

```
tacgcgcaga aaaaaaggat ctcaagaaga tcctttgatc ttttctacgg ggtctgacgc
tcagtggaac gaaaactcac gttaagggat tttggtcatg agattatcaa aaaggatctt
cacctagatc cttttaaatt aaaaatgaag ttttaaatca atctaaagta tatatgagta
aacttggtct gacagttacc aatgcttaat cagtgaggca cctatctcag cgatctgtct
atttcgttca tccatagttg cctgactccc cgtcgtgtag ataactacga tacgggaggg
cttaccatct ggccccagtg ctgcaatgat accgcgagac ccacgctcac cggctccaga
tttatcagca ataaaccagc cagccggaag ggccgagcgc agaagtggtc ctgcaacttt
atccgcctcc atccagtcta ttaattgttg ccgggaagct agagtaagta gttcgccagt
taatagtttg cgcaacgttg ttgccattgc tacaggcatc gtggtgtcac gctcgtcgtt
tggtatggct tcattcagct ccggttccca acgatcaagg cgagttacat gatcccccat
gttgtgcaaa aaagcggtta gctccttcgg tcctccgatc gttgtcagaa gtaagttggc
cgcagtgtta tcactcatgg ttatggcagc actgcataat tctcttactg tcatgccatc
cgtaagatgc ttttctgtga ctggtgagta ctcaaccaag tcattctgag aatagtgtat
gcggcgaccg agttgctctt gcccggcgtc aatacgggat aataccgcgc cacatagcag
aactttaaaa gtgctcatca ttggaaaacg ttcttcgggg cgaaaactct caaggatctt
accgctgttg agatccagtt cgatgtaacc cactcgtgca cccaactgat cttcagcatc
ttttactttc accagcgttt ctgggtgagc aaaaacagga aggcaaaatg ccgcaaaaaa
gggaataagg gcgacacgga aatgttgaat actcatactc ttcctttttc aatattattg
aagcatttat cagggttatt gtctcatgag cggatacata tttgaatgta tttagaaaaa
taaacaaata ggggttccgc gcacatttcc ccgaaaagtg ccacctgacg tc
```

Fig. 7

A

TCR beta chain

P2A linker

TCR alfa chain

Kozak

*Stu* I (1670)

*Sal*I (2)

T2A linker CD34t   *Bam* HI (2846)

TCR optimized

2850 bp

Fig. 7B

```
gtcgactgca cctacaccat gaagaccgtc actggccccc tgttcctgtg cttttggctg
cagctgaatt gcgtgtcccg aggcgagcag gtggagcaga gaccccccca cctgtcagtg
cgggagggcg atagcgccgt catcatttgc acatacactg accccaactc ctactatttc
ttttggtata aacaggaacc tgggggctgga ctgcagctgc tgatgaaggt gttcagctcc
actgagatta atgaaggcca ggggtttacc gtcctgctga acaagaaaga taagcagctg
agtctgaatc tgaccgcagc tcacccaggg gatagcgccg tgtacttctg cgctgtcaga
catgacagcg gatataacaa actgaccttt ggaaagggca cagtgctgct ggtctctcca
gacatccaga atcctgagcc agccgtgtac cagctgaagg accccaggtc tcaggatagc
accctgtgcc tgttcaccga ctttgattct cagattaacg tgcccaaaac catggaaagt
ggcaccttca tcacagacaa gactgtcctg gatatgaaag ccatggactc caagtctaac
ggggcaattg cctggagcaa tcagacctcc ttcacatgcc aggatatctt taaagagact
aatgcaacct atccttctag tgacgtgcca tgtgatgcca ccctgacaga gaagtccttc
gaaaccgaca tgaacctgaa ttttcagaac ctgtctgtga tgggcctgag aatcctgctg
ctgaaagtcg ccgggtttaa tctgctgatg acactgcgac tgtggtcaag caccggaggg
tccgggggcca caaacttctc tctgctgaag caggctggag atgtggagga aaatccagga
cctggaccag caactcgact gctgtgctac accgtgctgt gcctgctggg agcacgcatt
ctgaactcca aagtgatcca gacccccccga tatctggtca agggccaggg gcagaaagcc
aagatgcgat gcattcccga aagggacac cctgtggtct tctggtacca gcagaacaaa
aacaacgagt tcaagtttct gattaacttt cagaatcagg aagtgctgca gcagatcgat
atgaccgaga agcggttctc agccgaatgc cccagtaatt caccttgtag cctggagatc
cagtcctctg aagcaggcga ctccgccctg tatctgtgcg ccagttcact gtctaaggga
ggcttctacg agcagtattt tggacctggc acaagactga ctgtgctgga gacctgagg
aacgtgactc cccctaaagt ctctctgttt gagcctagta aggctgaaat cgcaaacaag
cagaaggcca ccctggtgtg cctggccaga ggcttctttc ccgatcacgt ggagctgagc
tggtgggtca acgggaaaga agtgcattca ggagtcagca cagaccctca agcctacaag
```

Fig. 7B

```
gagtccaatt actcttattg cctgagctcc cggctgcggg tgagcgccac cttctggcac
aaccccagga atcatttccg ctgtcaggtc cagtttcacg gactgtctga ggaagataaa
tggcctgagg gcagtcccaa gcctgtgacc cagaacattt cagctgaggc ctggggaagg
gctgactgtg gcatcacaag cgcatcctat catcagggcg tgctgtccgc caccatcctg
tacgagattc tgctgggcaa ggctacactg tatgcagtgc tggtctctgg gctggtgctg
atggctatgg tcaagaaaaa gaactctgag ttcgggagtg gagaaggccg agggagcctg
ctgacatgcg gcgatgtgga ggaaaatcct ggaccaccac gaggatggac cgccctgtgc
ctgctgtcac tgctgccaag cggctttatg tccctggaca acaatgggac tgcaacccca
gagctgccca cccagggcac attcagtaac gtgtcaacca atgtcagcta ccaggaaacc
acaactcctt ccacactggg atctactagt ctgcacccag tgagccagca tggcaacgag
gccaccacaa atattacaga aactaccgtg aagtttacat caactagcgt gatcacaagc
gtctatggca acactaattc tagtgtgcag agccagacat ccgtgatctc tactgtcttc
acaactccag ccaacgtgtc cacccccgag accacactga aaccatccct gtctcccggg
aatgtgagtg atctgtcaac taccagtact tcactggcaa cttctcctac caagccatac
acctcaagct cccctatcct gagcgacatc aaggccgaga tcaagtgctc cggaattcgc
gaagtgaaac tgacacaggg catctgcctg gagcagaaca agacttctag ttgtgctgag
tttaaaaagg atcgaggaga aggactggca cgggtgctgt gcggagagga acaggcagac
gctgatgcag gagcccaggt ctgcagtctg ctgctggctc agtcagaggt gcggccacag
tgtctgctgc tggtcctggc aaatagaacc gaaatttcaa gcaaactgca gctgatgaaa
aagcaccaga gcgacctgaa aaagctggga atcctggatt tcacagagca ggacgtggcc
agccatcaga gctactccca gaagaccctg attgctctgg tgacaagcgg cgccctgctg
gctgtcctgg gaatcaccgg atattttctg atgaatcggc ggagctggag cccaacaggc
gagagactgg aactggaacc ttgaggatcc
```

Fig. 8

A

pg1SAMEN-CMVSRa +TCR Telomerase optimized

7957 bp

*Sal* I (2142)

alfa Chain TCR

P2A peptide

beta Chain TCR

cloned TCR

*Stu* I (3810)

*Bam* HI (4986)

CD34t

T2A peptide

early CMV promoter

CMV

Fig. 8B

```
aggcgtatca cgaggccctt tcgtcttcaa gaattcatac cagatcaccg aaaactgtcc
tccaaatgtg tcccctcac actcccaaat tcgcgggctt ctgctcttag accactctac
cctattcccc acactcaccg gagccaaagc cgcggagcgc gttgacattg attattgact
agttattaat agtaatcaat tacggggtca ttagttcata gcccatatat ggagttccgc
gttacataac ttacggtaaa tggcccgcct ggctgaccgc ccaacgaccc ccgcccattg
acgtcaataa tgacgtatgt tcccatagta acgccaatag ggactttcca ttgacgtcaa
tgggtggagt atttacggta aactgcccac ttggcagtac atcaagtgta tcatatgcca
agtacgcccc ctattgacgt caatgacggt aaatggcccg cctggcatta tgcccagtac
atgaccttat gggactttcc tacttggcag tacatctacg tattagtcat cgctattacc
atggtgatgc ggttttggca gtacatcaat gggcgtggat agcggtttga ctcacgggga
tttccaagtc tccacccat tgacgtcaat gggagtttgt tttggcacca aaatcaacgg
gactttccaa aatgtcgtaa caactccgcc ccattgacgc aaatgggcgg taggcatgta
cggtgggagg tctatataag cagagctcaa taaaagagcc cacaacccct cactcggcgc
gccagtcttc cgatagactg cgtcgcccgg gtacccgtat tcccaataaa gcctcttgct
gtttgcatcc gaatcgtggt ctcgctgttc cttgggaggg tctcctctga gtgattgact
acccacgacg ggggtctttc atttgggggc tcgtccggga tttggagacc cctgcccagg
gaccaccgac ccaccaccgg gaggtaagct ggccagcaac ttatctgtgt ctgtccgatt
gtctagtgtc tatgtttgat gttatgcgcc tgcgtctgta ctagttagct aactagctct
gtatctggcg gacccgtggt ggaactgacg agttctgaac acccggccgc aacccaggga
gacgtcccag ggactttggg ggccgttttt gtggcccgac ctgaggaagg gagtcgatgt
ggaatccgac cccgtcagga tatgtggttc tggtaggaga cgagaaccta aaacagttcc
cgcctccgtc tgaatttttg ctttcggttt ggaaccgaag ccgcgcgtct tgtctgctgc
agcatcgttc tgtgttgtct ctgtctgact gtgtttctgt atttgtctga aaattagggc
cagactgtta ccactccctt aagtttgacc ttaggtcact ggaaagatgt cgagcggatc
gctcacaacc agtcggtaga tgtcaagaag agacgttggg ttaccttctg ctctgcagaa
tggccaacct ttaacgtcgg atggccgcga cacggcacct ttaaccgaga cctcatcacc
caggttaaga tcaaggtctt ttcacctggc ccgcatggac acccagacca ggtcccctac
atcgtgacct gggaagcctt ggcttttgac cccctccct gggtcaagcc ctttgtacac
cctaagcctc cgcctcctct tcctccatcc gccccgtctc tcccccttga acctcctcgt
tcgaccccgc ctcgatcctc cctttatcca gccctcactc cttctctagg cgcggaatt
cgcggccgtg acaagagtta ctaacagccc ctctctccaa gctcacttac aggctctcta
cttagtccag cacgaagtct ggagacctct ggcggcagcc taccaagaac aactggaccg
accggtggta cctcaccctt accgagtcgg cgacacagtg tgggtccgcc gacaccagac
taagaaccta gaacctcgct ggaaaggacc ttacacagtc ctgcagacca cccccaccgc
cctcaaagta gacggcatcg cagcttggat acacgccgcc cacgtgaagg ctgccgaccc
cggggggtgga ccatctctag actgacgcgg ccgctacgta gtcgactgca cctacaccat
gaagaccgtc actggccccc tgttcctgtg cttttggctg cagctgaatt gcgtgtcccg
aggcgagcag gtggagcaga gaccccccca cctgtcagtg cgggagggcg atagcgccgt
catcatttgc acatacactg acccaactc ctactatttc ttttggtata aacaggaacc
```

Fig. 8B

```
tggggctgga  ctgcagctgc  tgatgaaggt  gttcagctcc  actgagatta  atgaaggcca
ggggtttacc  gtcctgctga  acaagaaaga  taagcagctg  agtctgaatc  tgaccgcagc
tcacccaggg  gatagcgccg  tgtacttctg  cgctgtcaga  catgacagcg  gatataacaa
actgaccttt  ggaaagggca  cagtgctgct  ggtctctcca  gacatccaga  atcctgagcc
agccgtgtac  cagctgaagg  accccaggtc  tcaggatagc  accctgtgcc  tgttcaccga
ctttgattct  cagattaacg  tgcccaaaac  catggaaagt  ggcaccttca  tcacagacaa
gactgtcctg  gatatgaaag  ccatggactc  caagtctaac  ggggcaattg  cctggagcaa
tcagacctcc  ttcacatgcc  aggatatctt  taaagagact  aatgcaacct  atccttctag
tgacgtgcca  tgtgatgcca  ccctgacaga  gaagtccttc  gaaaccgaca  tgaacctgaa
ttttcagaac  ctgtctgtga  tgggcctgag  aatcctgctg  ctgaaagtcg  ccgggtttaa
tctgctgatg  acactgcgac  tgtggtcaag  caccggaggg  tccggggcca  caaacttctc
tctgctgaag  caggctggag  atgtggagga  aaatccagga  cctggaccag  caactcgact
gctgtgctac  accgtgctgt  gcctgctggg  agcacgcatt  ctgaactcca  aagtgatcca
gacccccga   tatctggtca  agggccaggg  gcagaaagcc  aagatgcgat  gcattcccga
gaagggacac  cctgtggtct  tctggtacca  gcagaacaaa  aacaacgagt  tcaagtttct
gattaacttt  cagaatcagg  aagtgctgca  gcagatcgat  atgaccgaga  agcggttctc
agccgaatgc  cccagtaatt  caccttgtag  cctggagatc  cagtcctctg  aagcaggcga
ctccgccctg  tatctgtgcg  ccagttcact  gtctaaggga  ggcttctacg  agcagtattt
tggacctggc  acaagactga  ctgtgctgga  agacctgagg  aacgtgactc  cccctaaagt
ctctctgttt  gagcctagta  aggctgaaat  cgcaaacaag  cagaaggcca  ccctggtgtg
cctggccaga  ggcttctttc  ccgatcacgt  ggagctgagc  tggtgggtca  acgggaaaga
agtgcattca  ggagtcagca  cagaccctca  agcctacaag  gagtccaatt  actcttattg
cctgagctcc  cggctgcggg  tgagcgccac  cttctggcac  aaccccagga  atcatttccg
ctgtcaggtc  cagtttcacg  gactgtctga  ggaagataaa  tggcctgagg  gcagtcccaa
gcctgtgacc  cagaacattt  cagctgaggc  ctggggaagg  gctgactgtg  gcatcacaag
cgcatcctat  catcagggcg  tgctgtccgc  caccatcctg  tacgagattc  tgctgggcaa
ggctacactg  tatgcagtgc  tggtctctgg  gctggtgctg  atggctatgg  tcaagaaaaa
gaactctgag  ttcgggagtg  gagaaggccg  agggagcctg  ctgacatgcg  gcgatgtgga
ggaaaatcct  ggaccaccac  gaggatggac  cgccctgtgc  ctgctgtcac  tgctgccaag
cggctttatg  tccctggaca  acaatgggac  tgcaacccca  gagctgccca  cccagggcac
attcagtaac  gtgtcaacca  atgtcagcta  ccaggaaacc  acaactcctt  ccacactggg
atctactagt  ctgcacccag  tgagccagca  tggcaacgag  gccaccacaa  atattacaga
aactaccgtg  aagtttacat  caactagcgt  gatcacaagc  gtctatggca  acactaattc
tagtgtgcag  agccagacat  ccgtgatctc  tactgtcttc  acaactccag  ccaacgtgtc
cacccccgag  accacactga  aaccatccct  gtctcccggg  aatgtgagtg  atctgtcaac
taccagtact  tcactggcaa  cttctcctac  caagccatac  acctcaagct  cccctatcct
gagcgacatc  aaggccgaga  tcaagtgctc  cggaattcgc  gaagtgaaac  tgacacaggg
```

Fig. 8B

```
catctgcctg  gagcagaaca  agacttctag  ttgtgctgag  tttaaaaagg  atcgaggaga
aggactggca  cgggtgctgt  gcggagagga  acaggcagac  gctgatgcag  gagcccaggt
ctgcagtctg  ctgctggctc  agtcagaggt  gcggccacag  tgtctgctgc  tggtcctggc
aaatagaacc  gaaatttcaa  gcaaactgca  gctgatgaaa  aagcaccaga  gcgacctgaa
aaagctggga  atcctggatt  tcacagagca  ggacgtggcc  agccatcaga  gctactccca
gaagaccctg  attgctctgg  tgacaagcgg  cgccctgctg  gctgtcctgg  gaatcaccgg
atattttctg  atgaatcggc  ggagctggag  cccaacaggc  gagagactgg  aactggaacc
ttgaggatcc  gataaaataa  aagattttat  ttagtctcca  gaaaaagggg  ggaatgaaag
accccacctg  taggtttggc  aagctagctt  aagtaacgcc  attttgcaag  gcatggaaaa
atacataact  gagaatagag  aagttcagat  caaggtcagg  aacagatgga  acagctgaat
atgggccaaa  caggatatct  gtggtaagca  gttcctgccc  cggctcaggg  ccaagaacag
atggaacagc  tgaatatggg  ccaaacagga  tatctgtggt  aagcagttcc  tgccccggct
cagggccaag  aacagatggt  ccccagatgc  ggtccagccc  tcagcagttt  ctagagaacc
atcagatgtt  tccagggtgc  cccaaggacc  tgaaatgacc  ctgtgcctta  tttgaactaa
ccaatcagtt  cgcttctcgc  ttctgttcgc  gcgcttctgc  tccccgagct  caataaaaga
gcccacaacc  cctcactcgg  ggcgccagtc  ctccgattga  ctgagtcgcc  cgggtacccg
tgtatccaat  aaaccctctt  gcagttgcat  ccgacttgtg  gtctcgctgt  tccttgggag
ggtctcctct  gagtgattga  ctacccgtca  gcgggggtct  ttcatttggg  ggctcgtccg
ggatcgggag  acccctgccc  agggaccacc  gacccaccac  cgggaggtaa  gctggctgcc
tcgcgcgttt  cggtgatgac  ggtgaaaacc  tctgacacat  gcagctcccg  gagacggtca
cagcttgtct  gtaagcggat  gccgggagca  gacaagcccg  tcagggcgcg  tcagcgggtg
ttggcgggtg  tcggggcgca  gccatgaccc  agtcacgtag  cgatagcgga  gtgtatactg
gcttaactat  gcggcatcag  agcagattgt  actgagagtg  caccatatgc  ggtgtgaaat
accgcacaga  tgcgtaagga  gaaaataccg  catcaggcgc  tcttccgctt  cctcgctcac
tgactcgctg  cgctcggtcg  ttcggctgcg  gcgagcggta  tcagctcact  caaaggcggt
aatacggtta  tccacagaat  caggggataa  cgcaggaaag  aacatgtgag  caaaaggcca
gcaaaaggcc  aggaaccgta  aaaaggccgc  gttgctggcg  ttttttccata ggctccgccc
ccctgacgag  catcacaaaa  atcgacgctc  aagtcagagg  tggcgaaacc  cgacaggact
ataaagatac  caggcgtttc  cccctggaag  ctccctcgtg  cgctctcctg  ttccgaccct
gccgcttacc  ggatacctgt  ccgcctttct  cccttcggga  agcgtggcgc  tttctcaatg
ctcacgctgt  aggtatctca  gttcggtgta  ggtcgttcgc  tccaagctgg  gctgtgtgca
cgaacccccc  gttcagcccg  accgctgcgc  cttatccggt  aactatcgtc  ttgagtccaa
cccggtaaga  cacgacttat  cgccactggc  agcagccact  ggtaacagga  ttagcagagc
gaggtatgta  ggcggtgcta  cagagttctt  gaagtggtgg  cctaactacg  gctacactag
aaggacagta  tttggtatct  gcgctctgct  gaagccagtt  accttcggaa  aaagagttgg
tagctcttga  tccggcaaac  aaaccaccgc  tggtagcggt  ggtttttttg  tttgcaagca
gcagattacg  cgcagaaaaa  aaggatctca  agaagatcct  ttgatctttt  ctacggggtc
tgacgctcag  tggaacgaaa  actcacgtta  agggattttg  gtcatgagat  tatcaaaaag
gatcttcacc  tagatccttt  taaattaaaa  atgaagtttt  aaatcaatct  aaagtatata
tgagtaaact  tggtctgaca  gttaccaatg  cttaatcagt  gaggcaccta  tctcagcgat
ctgtctattt  cgttcatcca  tagttgcctg  actccccgtc  gtgtagataa  ctacgatacg
```

Fig. 8B

```
ggagggctta  ccatctggcc  ccagtgctgc  aatgataccg  cgagacccac  gctcaccggc
tccagattta  tcagcaataa  accagccagc  cggaagggcc  gagcgcagaa  gtggtcctgc
aactttatcc  gcctccatcc  agtctattaa  ttgttgccgg  gaagctagag  taagtagttc
gccagttaat  agtttgcgca  acgttgttgc  cattgctgca  ggcatcgtgg  tgtcacgctc
gtcgtttggt  atggcttcat  tcagctccgg  ttcccaacga  tcaaggcgag  ttacatgatc
ccccatgttg  tgcaaaaaag  cggttagctc  cttcggtcct  ccgatcgttg  tcagaagtaa
gttggccgca  gtgttatcac  tcatggttat  ggcagcactg  cataattctc  ttactgtcat
gccatccgta  agatgctttt  ctgtgactgg  tgagtactca  accaagtcat  tctgagaata
gtgtatgcgg  cgaccgagtt  gctcttgccc  ggcgtcaaca  cgggataata  ccgcgccaca
tagcagaact  ttaaaagtgc  tcatcattgg  aaaacgttct  tcggggcgaa  aactctcaag
gatcttaccg  ctgttgagat  ccagttcgat  gtaacccact  cgtgcaccca  actgatcttc
agcatctttt  actttcacca  gcgtttctgg  gtgagcaaaa  acaggaaggc  aaaatgccgc
aaaaaaggga  ataagggcga  cacggaaatg  ttgaatactc  atactcttcc  tttttcaata
ttattgaagc  atttatcagg  gttattgtct  catgagcgga  tacatatttg  aatgtattta
gaaaaataaa  caaatagggg  ttccgcgcac  atttccccga  aaagtgccac  ctgacgtcta
agaaaccatt  attatcatga  cattaaccta  taaaaat
```

Fig. 9

Fig. 10

A

B

Fig. 11

Fig. 11

Fig. 12

Fig. 13

Fig. 14

Bone Marrow

Splean

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005116646 A

**Non-patent literature cited in the description**

- **UGEL S ; SCARSELLI E ; IEZZI M ; MENNUNI C ; PANNELLINI T ; CALVARUSO F ; CIPRIANI B ; DE PALMA R ; RICCI-VITIANI L ; PERANZONI E.** Autoimmune B-cell lymphopenia after successful adoptive therapy with telomerase-specific T lymphocytes. *Blood,* 2010
- **UGEL S ; SCARSELLI E ; IEZZI M ; MENNUNI C ; PANNELLINI T ; CALVARUSO F ; CIPRIANI B ; DE PALMA R ; RICCI-VITIANI L ; PERANZONI E.** Autoimmune B-cell lymphopenia after successful adoptive therapy with telomerase-specific T lymphocytes. *Blood,* 2010

- **UGEL S ; SCARSELLI E ; IEZZI M ; MENNUNI C ; PANNELLINI T ; CALVARUSO F ; CIPRIANI B ; DE PALMA R ; RICCI-VITIANI L ; PERANZONI E.** Autoimmune B-cell lymphopenia after successful adoptive therapy with telomerase-specific T lymphocytes. *Blood,* 2010, http://www.bloodjournal.org/content/115/7/1374.e-letters